# EUROPEAN PATENT APPLICATION

(11) **EP 4 744 482 A1**
(43) Date of publication of application: **20.05.2026**
(21) Application number: 24839834.9
(22) Date of filing: 04.07.2024
(51) Int. Cl.: A01K 67/30, A01K 67/35, C07K 19/00, C12N 15/09, C12N 15/12, C12N 15/62, C12P 21/02

(54) **TRANSGENIC LEPIDOPTERA INSECT**

(30) Priority: 12.07.2023 JP 2023114712
(71) Applicant: National Agriculture and Food Research Organization, Tsukuba-shi, Ibaraki 305-8517 (JP)
(72) Inventor: TATEMATSU Kenichiro, Tsukuba-shi, Ibaraki 305-8634 (JP)
(74) Representative: Mewburn Ellis LLP
(86) International application number: PCT/JP2024/080106
(87) International publication number: WO 2025/013948

(57) **Abstract**

The present invention addresses the objective of providing a novel expression system for producing a protein of interest stably and in large quantities in a lepidopteran insect such as a silkworm. The present invention also addresses the objective of providing a novel method for producing functional silk including a silk protein fused with a protein of interest. Provided is a transgenic lepidopteran insect, including a gene sequence of interest encoding a protein of interest or a fragment thereof in an exon sequence encoding a signal peptide or a functional fragment thereof of an endogenous gene, in which the protein of interest or the fragment thereof is fused to the C-terminal side of the signal peptide or the functional fragment thereof. Also provided is a transgenic lepidopteran insect, including a gene sequence of interest encoding a protein of interest or a fragment thereof in an exon sequence encoding a signal peptide or a functional fragment thereof of an endogenous gene, in which the protein of interest or the fragment thereof is fused between the signal peptide or the functional fragment thereof and a mature protein formed by cleaving the signal peptide from a precursor protein encoded by the endogenous gene or a C-terminal fragment thereof.

## Description

### Technical Field

The present invention relates to a transgenic lepidopteran insect, a method for producing the same, and a related technology.

### Background Art

The silk gland of the silkworm (*Bombyx mori*) has the ability to synthesize large quantities of protein in a short period of time. In addition, there is also an advantage in that the silk gland of the silkworm is easy to dissect because it is a large organ, and a synthesized protein is easy to collect because it is stored in the lumen of the silk gland. Therefore, a transgenic silkworm that expresses a protein of interest in the silk gland is regarded as a promising system for mass production of a protein.

The silk gland of the silkworm is a pair of organs located on the left and right sides, and each is composed of three regions: the anterior silk gland, the middle silk gland, and the posterior silk gland. Within a posterior silk gland cell, three major proteins that constitute fibroin, the fiber component of silk thread, are expressed: Fibroin H chain (hereinafter often abbreviated as "Fib H"), Fibroin L chain (hereinafter often abbreviated as "Fib L"), and fibrohexamerin (also called p25/FHX). Furthermore, within a middle silk gland cell, sericin, a gelatinous protein that is the coating component of silk thread, is expressed. The aforementioned three proteins expressed in a posterior silk gland cell form a complex (silk fibroin elementary unit; SFEU complex) in a ratio of Fib H: Fib L: p25 = 6:6:1, and are secreted into the lumen of the posterior silk gland. In contrast, sericin is secreted into the lumen of the middle silk gland after expression. The fibroin secreted into the lumen of the posterior silk gland subsequently transfers to the lumen of the middle silk gland, is coated with sericin, and is spun out as silk thread (Non-Patent Literature 1). Therefore, when using the silk gland of the silkworm as a protein expression system, a gene expression system that is specifically expressed in the middle silk gland or the posterior silk gland may be utilized.

When using the silk gland of the silkworm as a protein expression system, the GAL4/UAS system (Non-Patent Literature 2) and a mass expression method using a system that combines the sericin 1 promoter and the Hr3 enhancer (Non-Patent Literature 3) have been reported as recombinant protein expression systems. However, the GAL4/UAS system, which is superior in terms of protein expression level, is currently widely used.

The GAL4/UAS system is a gene regulation system that utilizes a combination of the transcription factor GAL4, derived from yeast, and the regulatory sequence UAS. For the GAL4/UAS system used as a protein production system in the silk gland of the silkworm, a GAL4 line in which the GAL4 gene is expressed under the control of a promoter of a gene specifically expressed in the middle or posterior silk gland, and a UAS line that expresses a protein gene of interest under the control of the regulatory sequence UAS are independently established by genetic modification using piggyBac, and then the two lines crossed to construct an expression system that expresses the protein of interest in the silk gland.

In the GAL4/UAS system, the need to cross the GAL4 line and the UAS line after the GAL4 line and the UAS line are separately established, which results in a time-consuming construction of the expression system, and the fact that the GAL4 gene and the UAS regulatory sequence are introduced at random positions in the genome, which can cause fluctuation in the expression level of the protein of interest, pose obstacles when establishing new GAL4 and UAS lines. Moreover, the expression levels achievable with the GAL4/UAS system are considered to have reached a technical limit.

Non-Patent Literature 4 discloses various methods implemented in the past to express transgenic proteins in the silk gland of the silkworm. However, previously reported methods have had problems such as low expression levels of fusion proteins and the loss of activity of proteins fused with silk proteins.

Accordingly, there is a need for new methods for producing proteins of interest stably and in large quantities, as well as for new methods for efficiently producing silk proteins fused with proteins of interest.

### Citation List

### Non-patent Literature

Non-patent Literature 1: Inoue S. et al., 2000, The Journal of Biological Chemistry, 275 (51): 40517-40528.
Non-patent Literature 2: Tatematsu K. et al., 2010, Transgenic Research, 19(3):473-87.
Non-patent Literature 3: Tomita M. et al., 2007, Transgenic Research, 16 (4):449-465.
Non-patent Literature 4: Tomita M. et al., 2011, Biotechnol Lett, 33:645-654.

### Summary of Invention

An object of the present invention is to provide a novel expression system for producing a protein of interest stably and in large quantities in a lepidopteran insect such as a silkworm. The present invention also addresses the objective of providing a novel method for producing functional silk comprising a silk protein fused with a protein of interest.

To solve the above objectives, the present inventors have conceived a novel expression system for expressing a gene of interest by utilizing the native promoter and enhancer activities of an endogenous gene. The expression system has been constructed by knocking-in a gene of interest encoding a protein of interest that is to be fused to the C-terminal side of an endogenous signal peptide, into an exon sequence encoding a signal peptide within a gene such as sericin or fibroin.

Generally, to efficiently knock-in an exogenous gene into the silkworm genome, it is necessary to cleave the target gene locus with a genome editing enzyme or the like. Therefore, the present inventors have designed a genome cleavage position within the exon sequence where the target gene sequence is to be introduced, and have attempted to knock-in the gene into this exon sequence. However, implementation of this method has resulted in 95% or more of the individuals in the injected generation failing to form normal cocoons, and 98% or more of the individuals failing to develop into adults capable of mating. Accordingly, it was found that establishing a line with this method was extremely difficult.

Therefore, instead of cleaving the genome within the exon sequence into which the gene sequence of interest is to be introduced, the present inventors attempted to knock-in the gene sequence of interest into the exon sequence by cleaving the genome within an intron sequence adjacent to the exon sequence. As a result, the present inventors have found that almost all individuals of the injected generation develop into adults with mating ability, and are able to produce the protein of interest in amounts greatly exceeding those of the conventional GAL4/UAS lines.

Furthermore, using the knock-in technology described above, the present inventors have introduced a fluorescent protein gene into an exon sequence of the endogenous fibroin gene, and created a cocoon comprising a fusion protein of the fluorescent protein and the full-length fibroin as a constituent fiber. As a result, under naked-eye observation in white light, the present inventors have succeeded in creating new fluorescent cocoons and fluorescent silk threads that exhibit overwhelmingly stronger coloration compared to fluorescent cocoons and silk threads exhibiting the strongest coloration from conventional technology, thereby completing the present invention. Based on the above research results, the present invention provides the following.
(1) A transgenic lepidopteran insect, comprising a gene sequence of interest encoding a protein of interest or a fragment thereof in an exon sequence encoding a signal peptide or a functional fragment thereof of an endogenous gene,
   wherein the protein of interest or the fragment thereof is fused to the C-terminal side of the signal peptide or the functional fragment thereof.
(2) The transgenic lepidopteran insect described in (1), wherein the endogenous gene encodes fibroin, sericin, and/or fibrohexamerin.
(3) The transgenic lepidopteran insect described in (2), wherein the fibroin is a fibroin H chain and/or a fibroin L chain.
(4) The transgenic lepidopteran insect described in (1), wherein the endogenous gene encodes:
   a fibroin H chain and a fibroin L chain;
   a fibroin H chain and sericin 1; or
   a fibroin H chain, a fibroin L chain, and sericin 1.
(5) The transgenic lepidopteran insect described in any one of (1) to (4), wherein the exon sequence comprises a transcription terminator sequence at the 3' end of the gene sequence of interest.
(6) A double-stranded circular DNA for introducing a gene sequence of interest into a genome cleavage position within an intron sequence of an endogenous gene of a transgenic lepidopteran insect, wherein
   the endogenous gene comprises:
      (a) a first spacer sequence adjacent to the 5' end of the genome cleavage position;
      (b) a second spacer sequence adjacent to the 3'-end side of the genome cleavage position;
      (c) a first recognition sequence recognized at the 5' end of the first spacer sequence by a first genome editing enzyme; and
      (d) a second recognition sequence recognized at the 3' end of the second spacer sequence by a second genome editing enzyme,
   the double-stranded circular DNA comprises, in this order:
      the first recognition sequence;
      the second spacer sequence;
      the first spacer sequence;
      a genome homologous sequence; and
      a gene sequence of interest,
   the genome homologous sequence consists of a base sequence homologous to a genome sequence from the second recognition sequence to an exon sequence located at the 3' end of the intron sequence or a partial sequence thereof, and
   the gene sequence of interest encodes a protein of interest or a fragment thereof which is fused to the C-terminal side of the signal peptide or the functional fragment thereof of the endogenous gene.
(7) A donor nucleic acid for producing a transgenic lepidopteran insect using a homologous recombination method, wherein
   the homologous recombination method comprises cleaving a genome cleavage position in an intron sequence in an endogenous gene by a genome editing enzyme,
   the donor nucleic acid comprises
      (a) a first genome homologous sequence and a second genome homologous sequence, derived from the endogenous gene, and
      (b) a gene sequence of interest placed therebetween,
   the first genome homologous sequence consists of a base sequence homologous to a genome sequence from a base positioned at the 5' end side of the genome cleavage position to an exon sequence positioned at the 3' end side of the intron sequence or a partial sequence thereof in the genome, and has a mutation in a recognition sequence of the genome editing enzyme,
   the second genome homologous sequence consists of a base sequence homologous to a genome sequence positioned at the 3' end side of the exon sequence or the partial sequence thereof in the genome, and
   the gene sequence of interest encodes a protein of interest or a fragment thereof which is fused to the C-terminal side of the signal peptide or the functional fragment thereof of the endogenous gene.
(8) A donor nucleic acid for producing a transgenic lepidopteran insect using a homologous recombination method, wherein
   the homologous recombination method comprises cleaving a genome cleavage position in an intron sequence in an endogenous gene by a genome editing enzyme,
   the donor nucleic acid comprises
      (a) a first genome homologous sequence and a second genome homologous sequence, derived from the endogenous gene, and
      (b) a gene sequence of interest placed therebetween,
   the first genome homologous sequence consists of a base sequence homologous to a genome sequence from a base positioned at the 5' end side of the intron sequence to an exon sequence positioned at the 5' end side of the intron sequence or a partial sequence thereof,
   the second genome homologous sequence consists of a base sequence homologous to a genome sequence from a base positioned at the 3' end side of the exon sequence or the partial sequence thereof and at the 5' end side of the genome cleavage position to a base positioned at the 3' end side of the genome cleavage position, and has a mutation in a recognition sequence of the genome editing enzyme, and
   the gene sequence of interest encodes a protein of interest or a fragment thereof which is fused to the C-terminal side of the signal peptide or the functional fragment thereof of the endogenous gene.
(9) The donor nucleic acid described in (7) or (8), comprising a nuclease recognition sequence at an end of the first genome homologous sequence and/or the second genome homologous sequence opposite to the gene sequence of interest.
(10) The donor nucleic acid described in (9), wherein the nuclease recognition sequence is a recognition sequence of the genome editing enzyme or a restriction enzyme recognition sequence.
(11) A method for producing a transgenic lepidopteran insect, comprising:
   the double-stranded circular DNA described in (6);
   the first genome editing enzyme or a nucleic acid encoding the first genome editing enzyme in a state which allows for its expression; and
   the second genome editing enzyme or a nucleic acid encoding the second genome editing enzyme in a state which allows for its expression,
   into an egg of a lepidopteran insect by a microinjection method.
(12) A method for producing a transgenic lepidopteran insect, comprising:
   the donor nucleic acid described in any one of (7) to (10); and
   the genome editing enzyme or a nucleic acid encoding the genome editing enzyme in a state which allows for its expression, into an egg of a lepidopteran insect by a microinjection method.
(13) A method for producing the protein of interest or the fragment thereof using the transgenic lepidopteran insect described in any one of (1) to (5) or a transgenic lepidopteran insect produced by the method described in (11) or (12).
(14) The method described in (13), wherein the lepidopteran insect is a silk spinning insect, and the protein of interest or the fragment thereof is produced in the silk gland of the silk spinning insect.

The present invention further provides the following.
(1) A transgenic lepidopteran insect, comprising a gene sequence of interest encoding a protein of interest or a fragment thereof in an exon sequence encoding a signal peptide or a functional fragment thereof of an endogenous gene,
   wherein the protein of interest or the fragment thereof is fused between the signal peptide or the functional fragment thereof and a mature protein formed by cleaving the signal peptide from a precursor protein encoded by the endogenous gene or a C-terminal fragment thereof.
(2) The transgenic lepidopteran insect described in (1), wherein the mature protein is fibroin, sericin, and/or fibrohexamerin.
(3) The transgenic lepidopteran insect described in (2), wherein the fibroin is a fibroin H chain and/or a fibroin L chain.
(4) The transgenic lepidopteran insect described in any one of (1) to (3), wherein the protein of interest is selected from the group consisting of a fluorescent protein, an antibody, an antigenic polypeptide, an enzyme, a cytokine, and an antimicrobial polypeptide.
(5) The transgenic lepidopteran insect described in any one of (1) to (4), which homozygously comprises the exon sequence comprising the gene sequence of interest.
(6) A donor nucleic acid for producing a transgenic lepidopteran insect using a homologous recombination method, wherein
   the homologous recombination method comprises cleaving a genome cleavage position in an intron sequence in an endogenous gene by a genome editing enzyme,
   the donor nucleic acid comprises
      (a) a first genome homologous sequence and a second genome homologous sequence, derived from the endogenous gene, and
      (b) a gene sequence of interest placed therebetween,
   the first genome homologous sequence consists of a base sequence homologous to a genome sequence from a base positioned at the 5' end side of the genome cleavage position to an exon sequence positioned at the 3' end side of the intron sequence or a partial sequence thereof in the genome, and has a mutation in a recognition sequence of the genome editing enzyme,
   the second genome homologous sequence consists of a base sequence homologous to a genome sequence positioned at the 3' end side of the exon sequence or the partial sequence thereof in the genome, and
   the gene sequence of interest encodes a protein of interest or a fragment thereof which is fused between the signal peptide or the functional fragment thereof of the endogenous gene and a mature protein formed by cleaving the signal peptide from a precursor protein encoded by the endogenous gene or a C-terminal fragment thereof.
(7) A donor nucleic acid for producing a transgenic lepidopteran insect using a homologous recombination method, wherein
   the homologous recombination method comprises cleaving a genome cleavage position in an intron sequence in an endogenous gene by a genome editing enzyme,
   the donor nucleic acid comprises
      (a) a first genome homologous sequence and a second genome homologous sequence, derived from the endogenous gene, and
      (b) a gene sequence of interest placed therebetween,
   the first genome homologous sequence consists of a base sequence homologous to a genome sequence from a base positioned at the 5' end side of the intron sequence to an exon sequence positioned at the 5' end side of the intron sequence or a partial sequence thereof,
   the second genome homologous sequence consists of a base sequence homologous to a genome sequence from a base positioned at the 3' end side of the exon sequence or the partial sequence thereof and at the 5' end side of the genome cleavage position to a base positioned at the 3' end side of the genome cleavage position, and has a mutation in a recognition sequence of the genome editing enzyme, and
   the gene sequence of interest encodes a protein of interest or a fragment thereof which is fused between the signal peptide or the functional fragment thereof of the endogenous gene and a mature protein formed by cleaving the signal peptide from a precursor protein encoded by the endogenous gene or a C-terminal fragment thereof.
(8) The donor nucleic acid described in (6) or (7), comprising a nuclease recognition sequence at an end of the first genome homologous sequence and/or the second genome homologous sequence opposite to the gene sequence of interest.
(9) The donor nucleic acid described in (8), wherein the nuclease recognition sequence is a recognition sequence of the genome editing enzyme or a restriction enzyme recognition sequence.
(10) A method for producing a transgenic lepidopteran insect, comprising:
   an introduction step of introducing a donor nucleic acid described in (6) or (7); and
   the genome editing enzyme or a nucleic acid encoding the genome editing enzyme in a state which allows for its expression,
   into an egg of a lepidopteran insect by a microinjection method.
(11) A method for producing a fusion protein comprising the protein of interest or the fragment thereof and the mature protein or the C-terminal fragment thereof, using the transgenic lepidopteran insect described in any one of (1) to (5) or a transgenic lepidopteran insect produced by the method described in (10).
(12) The method described in (11), wherein the lepidopteran insect is a silk spinning insect, and the fusion protein is produced in the silk gland of the silk spinning insect.
(13) A fusion protein comprising:
   a protein of interest or a fragment thereof; and
   fibroin, sericin, or fibrohexamerin,
   in this order from the N-terminal side.
(14) The fusion protein described in (13), wherein the fibroin is a fibroin H chain and/or a fibroin L chain.
(15) A cocoon or silk thread comprising the fusion protein described in (13) or (14).
(16) A cocoon or silk thread, wherein one or more silk proteins selected from the group consisting of fibroin H chain, fibroin L chain, sericin 1, sericin 2, sericin 3, and fibrohexamerin contained in the cocoon or silk thread consist of the fusion protein described in (13) or (14).
(17) The cocoon or silk thread described in (16), which is derived from the transgenic lepidopteran insect described in any one of (1) to (5), or a transgenic lepidopteran insect produced by the method described in (10).
(18) The cocoon or silk thread described in (16) or (17), wherein the protein of interest is selected from the group consisting of a fluorescent protein, an antibody, an antigenic polypeptide, an enzyme, a cytokine, and an antimicrobial polypeptide.

The present specification encompasses the disclosure of JP 2023-114712A, to which the present application claims the priority.

### Brief Description of Drawing

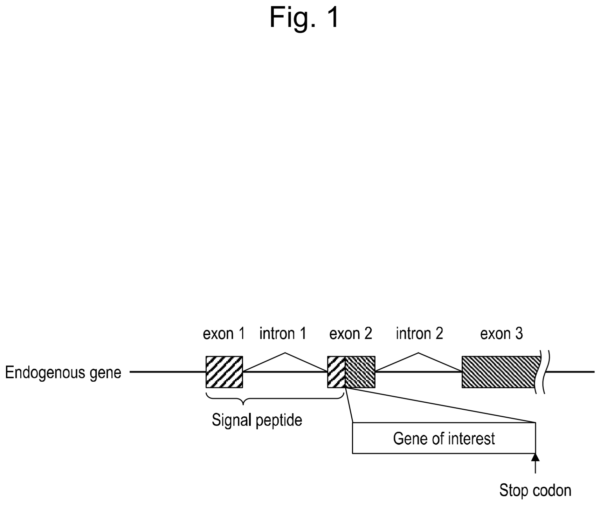

[Figure 1] Figure 1 shows the introduction of a gene sequence of interest having a stop codon into an endogenous gene. The gene sequence of interest is introduced into an exon sequence that encodes a signal peptide in the endogenous gene. The protein of interest encoded by the gene sequence of interest is fused to the C-terminal side of the signal peptide encoded by the endogenous gene.

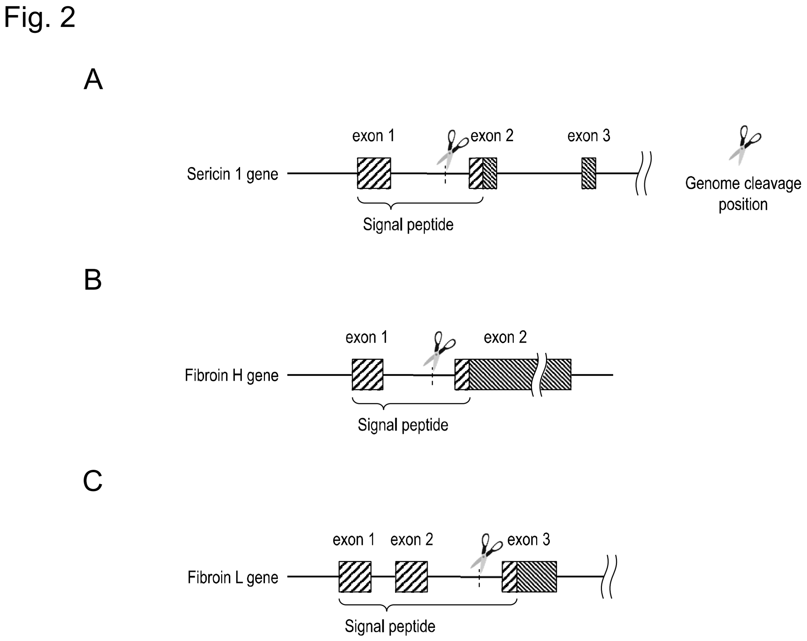

[Figure 2] Figure 2 shows the genome cleavage position in an endogenous gene for the production of a knock-in line. The genome cleavage position is designed within an intron sequence located at the 5' end of the exon sequence into which the gene sequence of interest is to be introduced. As examples of the endogenous gene into which the gene sequence of interest is to be introduced, Figure 2A shows the sericin 1 gene, Figure 2B shows the fibroin H gene, and Figure 2C shows the fibroin L gene.

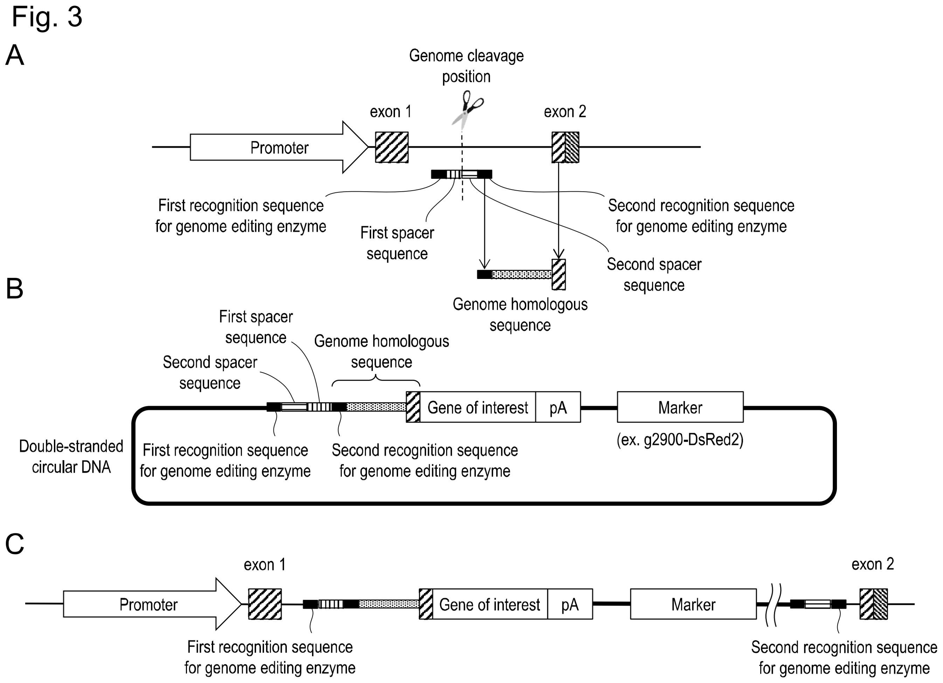

[Figure 3] Figure 3 shows an overview of gene knock-in using the TAL-PITCh method. Figure 3A shows the locations in the endogenous gene from which each sequence used to construct the donor nucleic acid for the TAL-PITCh method is derived. Figure 3B shows the structure of the double-stranded circular DNA used for the TAL-PITCh method. Figure 3C shows the structure of a knock-in gene obtained by knocking-in a gene sequence of interest using the TAL-PITCh method.

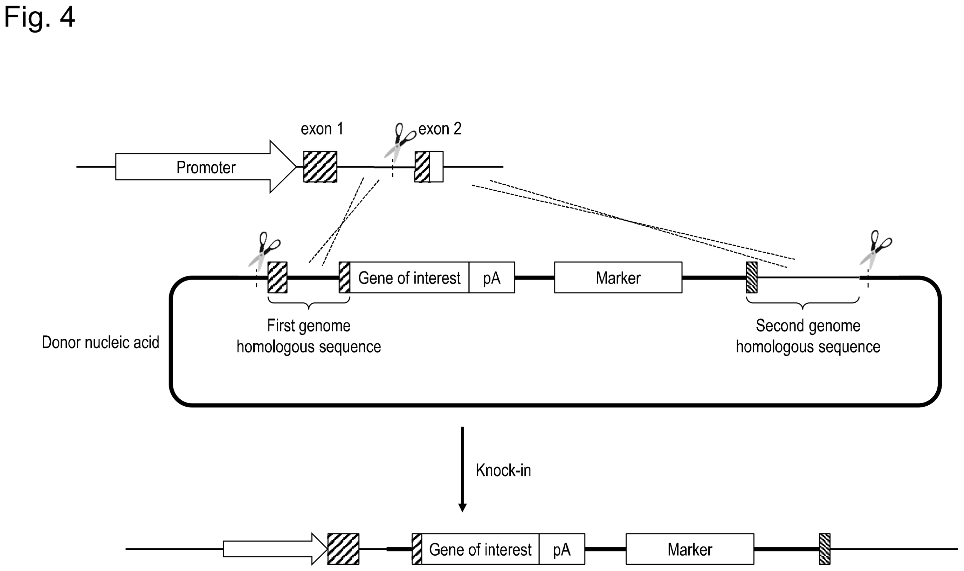

[Figure 4] Figure 4 shows a method of gene knock-in using the homologous recombination method.

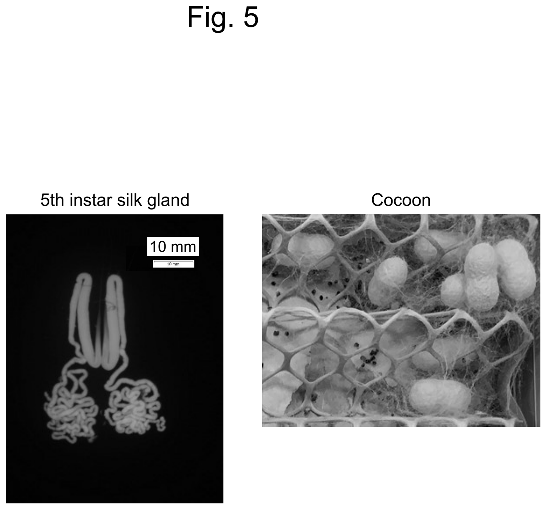

[Figure 5] Figure 5 shows the observation results of silk glands and cocoons in 5th instar larvae of the SP(FibH)-EGFP knock-in line.

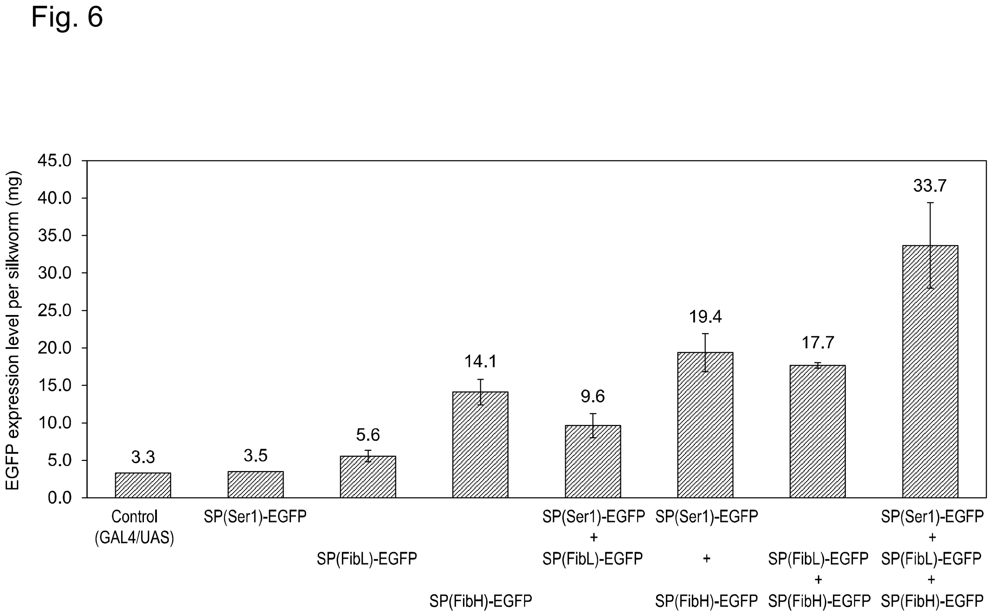

[Figure 6] Figure 6 shows the results of measuring the EGFP expression level per silkworm for each knock-in line. The graph shows the average of measured values for n = 1 to 4, and the error bars indicate the standard error.

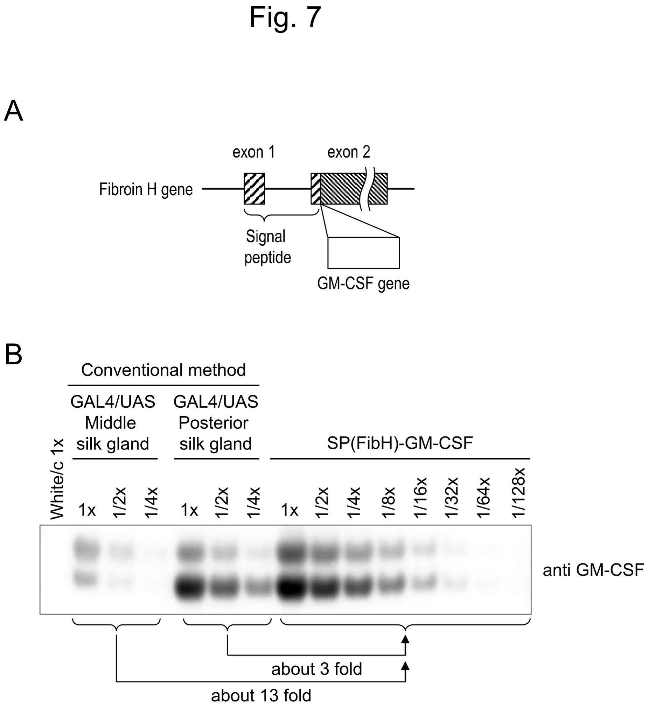

[Figure 7] Figure 7 shows GM-CSF production in a silkworm line in which the GM-CSF gene sequence was knocked-in to the second exon of the endogenous fibroin H gene. Figure 7A shows the knock-in of the GM-CSF gene sequence into the fibroin H gene. Figure 7B shows the results of detecting GM-CSF by Western blotting.

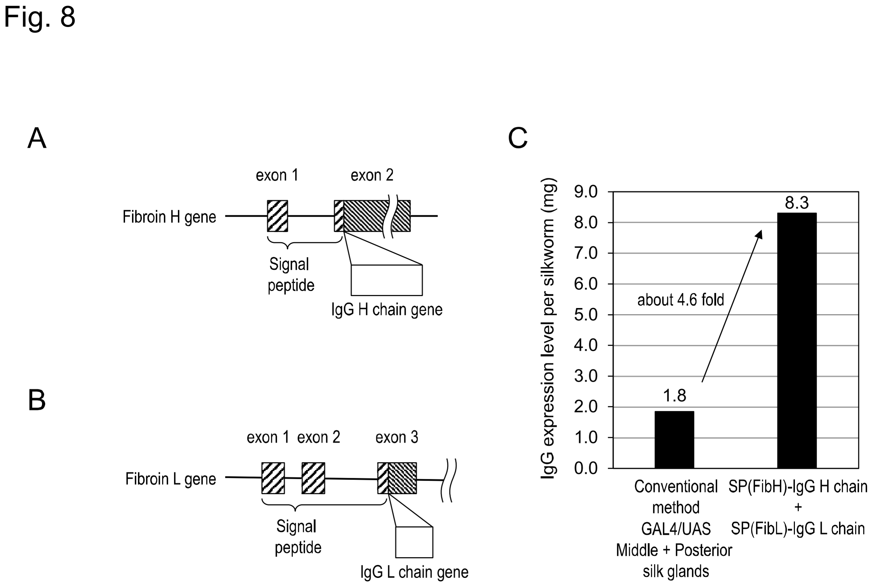

[Figure 8] Figure 8 shows the IgG production in a silkworm line in which gene sequences encoding the IgG H chain and IgG L chain are knocked-in to the second exon of the endogenous fibroin H gene and the third exon of the endogenous fibroin L gene, respectively. Figure 8A shows the knock-in of the IgG H chain gene sequence into the fibroin H gene. Figure 8B shows the knock-in of the IgG L chain gene sequence into the fibroin L gene. Figure 8C shows the IgG production amount.

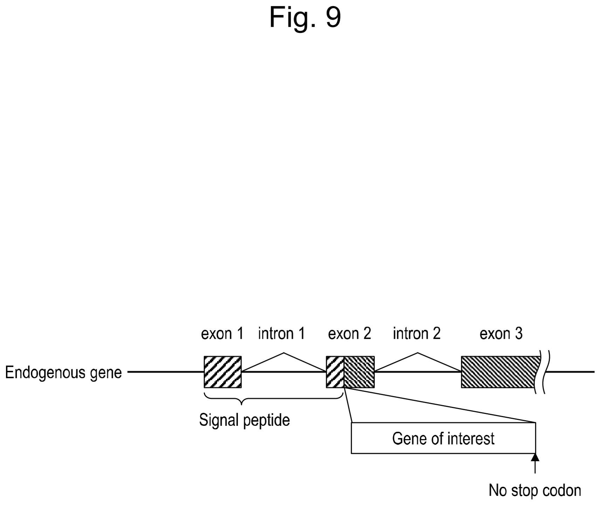

[Figure 9] Figure 9 shows the introduction of a gene sequence of interest without a stop codon into an endogenous gene. The gene sequence of interest is introduced into an exon sequence that encodes a signal peptide in the endogenous gene. The protein of interest encoded by the gene sequence of interest is fused between a secretory peptide and a mature protein that is formed by cleaving the signal peptide from a precursor protein encoded by the endogenous gene.

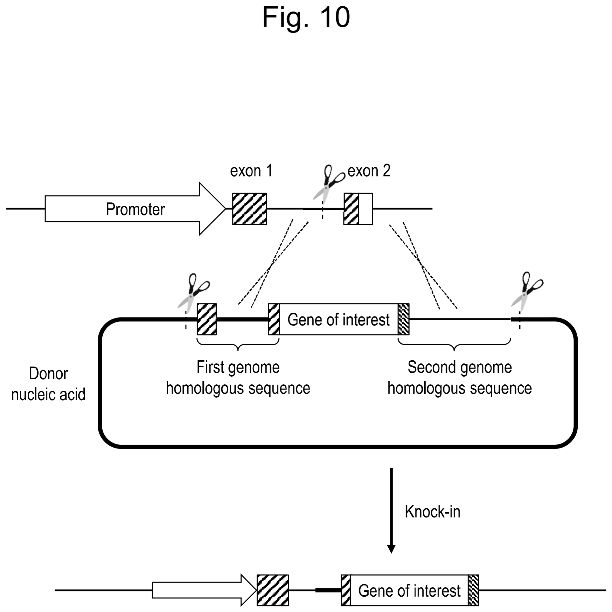

[Figure 10] Figure 10 shows a method of gene knock-in using the homologous recombination method.

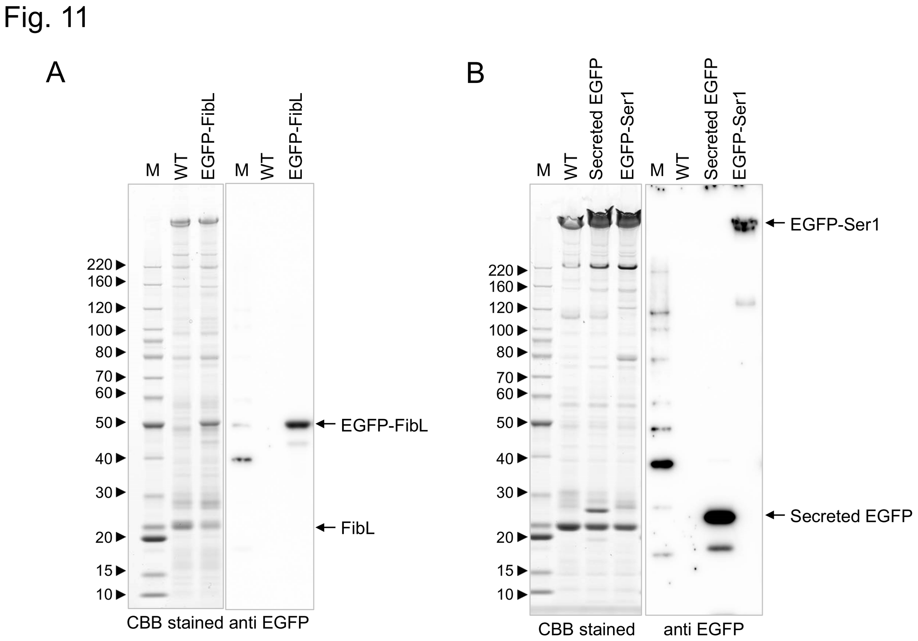

[Figure 11] Figure 11 shows the results of detecting the EGFP protein by Western blotting. Figure 11A shows the results for the wild-type line (WT) and the EGFP-FibL knock-in line. Figure 11B shows the results for the wild-type line (WT), the SP(Ser1)-EGFP knock-in line, and the EGFP-Ser1 knock-in line.

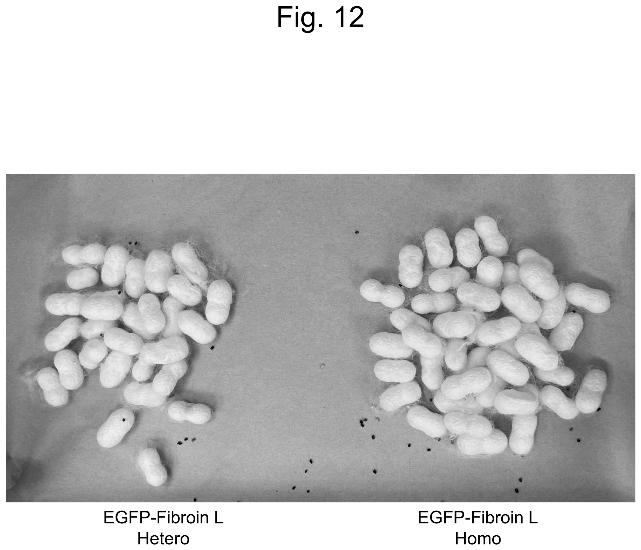

[Figure 12] Figure 12 shows a photograph of cocoons obtained from the EGFP-FibL knock-in line that heterozygously or homozygously comprises the EGFP-FibL knock-in gene, taken under normal white light.

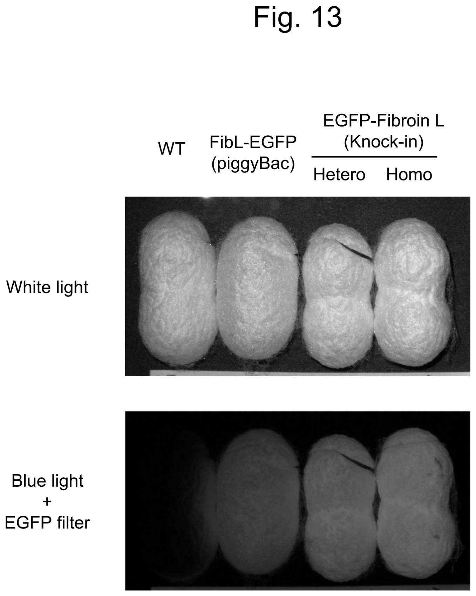

[Figure 13] Figure 13 shows a photograph of cocoons produced by heterozygotes and homozygotes of the EGFP-FibL knock-in line produced by the method of the present invention, as well as cocoons produced using the conventional piggyBac system, observed under white light or by fluorescence observation.

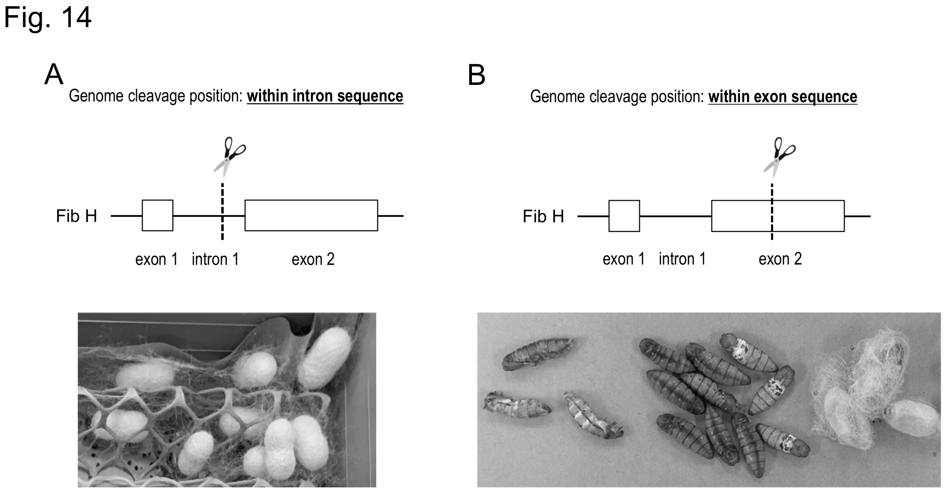

[Figure 14] Figure 14 shows the results of performing knock-in by designing the genome cleavage position within an intron sequence or an exon sequence. Figure 14A shows the results of performing homologous recombination via genome cleavage in an intron sequence of the Fib H gene. 99% or more of the individuals developed into adults with mating ability, and no defective cocoon formation or defective mating was observed. Figure 14B shows the results of performing homologous recombination via genome cleavage in an exon sequence of the Fib H gene. Less than 2% of the individuals developed into adults with mating ability, and 95% or more of the injected generation exhibited pupation failure, naked pupae, or thin cocoons.

### Description of Embodiments

### 1. Transgenic lepidopteran insect

### 1-1. Outline

The first aspect of the present invention is a transgenic lepidopteran insect. The transgenic lepidopteran insect of the present invention comprises a gene sequence of interest in an exon sequence encoding a signal peptide or a functional fragment thereof of an endogenous gene, and expresses a protein of interest or a fragment thereof which is fused to the C-terminal side of the signal peptide or the functional fragment thereof. The transgenic lepidopteran insect of this aspect can produce the protein of interest stably and in large quantities.

### 1-2. Definition

The following terms that are often used herein will be defined.

In this specification, a "lepidopteran insect" refers to an insect belonging to the taxonomic order *Lepidoptera,* that is a butterfly or a moth. Examples of the butterfly includes insects belonging to the families *Nymphalidae, Papilionidae, Pieridae, Lycaenidae,* and *Hesperiidae.* Examples of the moth include insects belonging to the families *Saturniidae, Bombycidae, Brahmaeidae, Eupterotidae, Lasiocampidae, Psychidae, Geometridae, Arctiidae, Noctuidae, Pyralidae, Sphingidae,* and the like. For example, examples of the moth include species belonging to the genera *Bombyx, Samia, Antheraea, Saturnia, Attacus,* and *Rhodinia,* and specifically include silkworms (*Bombyx mori*), *Bombyx mandarina, Samia cynthia* (including *Samia cynthia ricini* and hybrids between *Samia cynthia* and *Samia cynthia ricini*), *Antheraea yamamai, Antheraea pernyi, Saturnia japonica, Actias gnoma,* and the like. The lepidopteran insect as a host for the transformant of the present invention is not limited to these, but silkworms, which have high industrial applicability, are particularly preferable as a host.

A "transgenic lepidopteran insect" refers to a lepidopteran insect that has been genetically modified to carry foreign DNA produced using genetic modification technology, or a progeny thereof. The transgenic lepidopteran insect as used herein particularly means a genetically modified organism obtained by introducing foreign DNA into an egg of a lepidopteran insect by a microinjection method.

As used herein, a "silk gland" is a tubular organ that is a modified salivary gland and has the functions of producing, accumulating, and secreting liquid silk. Silk glands are usually found as a pair on either side along the digestive tract of insects capable of spinning silk thread, mainly during the larval stage, and each silk gland is composed of three regions: the anterior, middle, and posterior silk glands. The posterior silk gland produces and secretes fibroin, which is the fiber component of silk thread. The middle silk gland produces and secretes sericin, which is the coating component, and accumulates sericin in its lumen along with the fibroin that has been transferred from the posterior silk gland.

As used herein, an "endogenous gene" refers to a gene derived from a lepidopteran insect that is congenitally present on the genome of the lepidopteran insect. In the present invention, the endogenous gene is, in principle, a gene that encodes a protein having a signal peptide. Therefore, as used herein, the endogenous gene is, in principle, a gene that encodes a secretory protein or a membrane protein. The secretory protein may be, for example, any protein that constitutes silk thread. As used herein, any protein constituting silk thread is often referred to as a "silk protein," and a gene encoding a silk protein is referred to as a "silk gene." Specific examples of the endogenous gene of lepidopteran insects include the genes encoding fibroin, sericin, and fibrohexamerin.

Furthermore, as used herein, an "exogenous gene" or a "foreign gene" refers to a foreign gene that is acquired exogenously and postnatally through artificial manipulation or the like and does not exist in the genome of a wild-type lepidopteran insect.

"Fibroin" is a protein that constitutes the fiber component of silk thread. The fibroin of the silkworm is mainly composed of three proteins: Fibroin H chain (Fib H), Fibroin L chain (Fib L), and Fibrohexamerin. As mentioned above, fibrohexamerin is also called p25/FHX.

"Sericin" is a protein that covers, in a layered manner, the outside of the fibers formed by fibroin in silk thread. In silkworms, sericin is synthesized within middle silk gland cells and after that secreted into the lumen of middle silk glands. The functions of sericin are known to include adhering fibroin fibers to each other, as well as protecting fibroin fibers from external stimuli. Although silkworms can spin thread from the stage immediately after hatching, the protein components of the silk thread spun at each instar and the silk thread of the cocoon are different, and the variant composition of sericin contained therein is also different. Generally, in silkworms, about six types of sericin protein variants (Sericin 1A', Sericin 1C, Sericin 1D, Sericin 2, Sericin 3, and Sericin 4) biosynthesized from four types of sericin genes (Ser1, Ser2, Ser3, and Ser4) are known. Among these, the four main sericin variants contained in cocoons are Sericin 1A', Sericin 1C, Sericin 1D, and Sericin 3. When simply referred herein to as "sericin", it shall mean the general term for sericin unless otherwise specified.

As used herein, a "signal peptide" or a "secretory signal" refers to an extracellular transport signal required for secreting a protein biosynthesized by gene expression to the outside of the cell. After translation, the signal peptide is cleaved and removed by a signal peptidase before being secreted outside the cell. As used herein, the signal peptide is often abbreviated as "SP," and the name of the endogenous gene from which the signal peptide is derived is indicated in parentheses. A signal peptide is typically a relatively short peptide sequence of several tens of amino acids or less and is characterized by a sequence that is rich in hydrophobicity. The sequence of a signal peptide can be predicted based on the amino acid sequence of the protein using a prediction tool such as SignalP. It is also possible to use structural predictions provided in databases. For example, in the case of the silkworm, the sequence region of the signal peptide can also be determined based on the sequence annotations provided in databases such as KAIKObase and KAIKOcDNA, which are available in the Agrigenomics Information Database.

As used herein, a "functional fragment" of a signal peptide refers to a fragment that consists of a partial sequence of the signal peptide and retains the extracellular transport signal activity. For example, the functional fragment of a signal peptide may retain 50% or more, 60% or more, 70% or more, 80% or more, 90% or more, 95% or more, or 98% or more, or an equivalent or greater level of the extracellular transport signal activity of the full-length signal peptide. The amino acid length of the functional fragment is not particularly limited as long as the activity of the full-length signal peptide is retained, and may be, for example, 50% or more, 60% or more, 70% or more, 80% or more, 90% or more, 95% or more, or 98% or more of the full length.

As used herein, "full-length" means the entire amino acid sequence corresponding to a protein that is synthesized and functions in vivo, or the entire base sequence of the gene that encodes the protein. In principle, in the case of a gene, the sequence from the start codon to the stop codon corresponds to the full-length gene, and in the case of a protein, the polypeptide or peptide consisting of the amino acid sequence encoded by the full-length gene corresponds to the full-length protein. However, in the case of a secretory protein, the endogenous signal peptide contained at the N-terminal side is cleaved and removed during the secretion process and thus is ultimately not included. Therefore, in the case of a secretory protein, "full-length" does not necessarily have to include the signal peptide. As used herein, of the full-length proteins, the one before the signal peptide is cleaved and removed during the secretion process is referred to as a "precursor protein," and the one after the signal peptide has been cleaved and removed is referred to as a "mature protein" to distinguish between them.

As used herein, an "exon" means a region of the base sequence of a gene that remains in the mature transcript. Generally, in eukaryotes, a gene is transcribed into a primary transcript, which is then spliced to remove intervening regions called "introns", after which the exons are joined together to form a mature transcript. As used herein, an "exon sequence" means a base sequence corresponding to an exon, and an "intron sequence" means a base sequence corresponding to an intron. For any gene, the exon and intron sequences can be determined by comparing the genome sequence of the gene with its cDNA sequence. It is also possible to obtain sequence information published in databases such as that of the National Center for Biotechnology Information (NCBI) or to predict the exon/intron structure using genome analysis tools available in the art. For example, for gene information of the silkworm, the exon and intron sequences can be searched for using databases such as KAIKObase and KAIKOcDNA, which are available from the Agrigenomics Information Database.

As used herein, a "gene sequence of interest" refers to a gene sequence that encodes a protein of interest or a fragment thereof. The gene sequence of interest may be a sequence of a gene derived from a genome or a gene consisting of cDNA, and may or may not comprise an intron within the gene sequence of interest. The gene sequence of interest may or may not further comprise a stop codon in addition to the gene sequence encoding the protein of interest or a fragment thereof, and may or may not comprise a transcription terminator sequence downstream of the stop codon.

As used herein, a "protein of interest" is a desired protein encoded by a gene of interest. The type of the protein of interest is not particularly limited. The protein of interest may be either a structural protein or a functional protein. Examples of the structural protein include fibrous proteins such as collagen, actin, myosin, and fibroin, as well as keratin and histones. Examples of the functional protein include peptide hormones (such as insulin, calcitonin, parathormones, and growth hormones), cytokines (such as granulocyte-macrophage colony-stimulating factor (GM-CSF), epidermal growth factor (EGF), fibroblast growth factor (FGF), interleukins (IL), interferons (IFN), tumor necrosis factor α (TNF-α), and transforming growth factor β (TGF-β)), transcription factors (including GAL4), antibodies (such as immunoglobulins), serum albumin, hemoglobin, enzymes, fluorescent proteins, pigment-synthesizing proteins, and luminescent proteins. The immunoglobulins may be of any class (e.g., IgG, IgE, IgM, IgA, IgD, and IgY) or of any subclass (e.g., IgG1, IgG2, IgG3, IgG4, IgA1, and IgA2). The fluorescent protein is not limited and may be, for example, CFP, AmCyan, RFP, DsRed, YFP, or GFP (including a derivative such as EGFP or EYFP). The pigment-synthesizing proteins may be, for example, a protein involved in the biosynthesis of melanin-based pigments (including dopamine melanin), ommochrome-based pigments, or pteridine-based pigments. The luminescent protein may be, for example, aequorin or luciferase. The protein of interest may be either a wild-type protein or a variant protein.

As used herein, a "fragment" of a protein refers to a polypeptide or peptide that comprises a partial region of the full-length protein. The fragment preferably retains the activity. For example, the fragment may retain 50% or more, 60% or more, 70% or more, 80% or more, 90% or more, 95% or more, 98% or more, or an equivalent or greater level of the activity of the full-length protein. The amino acid length of the fragment is not particularly limited as long as the activity of the full-length protein is retained, and may be, for example, 50% or more, 60% or more, 70% or more, 80% or more, 90% or more, 95% or more, or 98% or more of the full length.

As used herein, a "transcription terminator sequence" is a sequence capable of terminating gene transcription and is also called a terminator. The type of the transcription terminator sequence is not particularly limited. Preferably, the transcription terminator sequence is a terminator derived from the same biological species as the transgenic lepidopteran insect. For example, in the case of insects such as silkworms, an hsp70 terminator or a SV40 terminator can be used.

As used herein, "a plurality" refers to an integer of 2 or more, for example, an integer of 2 to 10, 2 to 7, 2 to 5, 2 to 4, or 2 to 3.

As used herein, the "identity" of a base sequence refers to the percentage (%) of the number of matching bases to the entire length of the base sequence when two base sequences to be compared are aligned after inserting gaps into one or both of them as necessary to maximize the number of matching bases.

As used herein, a "homologous sequence" refers to a base sequence having an identity of about 60% or more to a reference sequence. The identity of the homologous sequence to the reference sequence may be, for example, 70% or more, 80% or more, 90% or more, 95% or more, 96% or more, 97% or more, 98% or more, 99% or more, or 99.9% or more. A "genome homologous sequence" indicates a base sequence having any of the above-mentioned identities when the genome sequence of a lepidopteran insect is used as the reference sequence, and a "genome sequence" refers to a sequence having 100% identity to the corresponding base sequence on the genome.

As used herein, an "amino acid identity" refers to the percentage (%) of the number of matching amino acid residues to the number of the entire amino acid residues when two polypeptide amino acid sequences to be compared are aligned after inserting gaps into one or both of them as necessary to maximize the number of matching residues.

As used herein, "substitution of an amino acid" refers to a substitution among the 20 types of amino acids that constitute natural proteins, wherein the substitution occurs within a group of conservative amino acids having similar properties such as charge, side chain, polarity, or aromaticity. Examples include substitution within the following groups: the uncharged polar amino acid group with low-polarity side chains (Gly, Asn, Gln, Ser, Thr, Cys, and Tyr), the branched-chain amino acid group (Leu, Val, and Ile), the neutral amino acid group (Gly, Ile, Val, Leu, Ala, Met, and Pro), the neutral amino acid group with hydrophilic side chains (Asn, Gln, Thr, Ser, Tyr, and Cys), the acidic amino acid group (Asp and Glu), the basic amino acid group (Arg, Lys, and His), and the aromatic amino acid group (Phe, Tyr, and Trp).

As used herein, unless otherwise specified, the "5'-end side" and the "3'-end side" defines directionality based on the 5'-end side and 3'-end side, respectively, of the transcript transcribed from the endogenous gene. Furthermore, as used herein, unless otherwise specified, "upstream" and "downstream" indicate the upstream and downstream directions of the gene, respectively, based on the direction of transcription of the endogenous gene.

### 1-3. Configuration

The transgenic lepidopteran insect of the present invention comprises a gene sequence of interest encoding a protein of interest or a fragment thereof in an exon sequence encoding a signal peptide or a functional fragment thereof of an endogenous gene. The gene sequence of interest is contained in the exon sequence such that the protein of interest or a fragment thereof is fused to the C-terminal side of the signal peptide or a functional fragment thereof.

As used herein, an "exon sequence encoding a signal peptide or a functional fragment thereof of an endogenous gene" (hereinafter often referred to as "target exon sequence") is not limited as long as it is an exon sequence that encodes a signal peptide in an endogenous gene. Typically, in an endogenous gene, a signal peptide is encoded by the first exon located at the most upstream position in the mRNA transcribed from the endogenous gene, or by a plurality of exon sequences including the first exon; however, the target exon sequence may be any exon sequence. For example, the target exon sequence may be the first exon, the second exon, the third exon, or the fourth exon. The target exon sequence may be, for example, an exon that encodes the C-terminal amino acid residue of the signal peptide, or an exon adjacent to the 5'-end side thereof.

In the transgenic lepidopteran insect of the present invention, the gene sequence of interest is inserted into the target exon sequence such that the protein of interest or a fragment thereof, which is encoded by the gene sequence of interest, is fused to the C-terminal side of the signal peptide or the functional fragment thereof of the endogenous gene. More specifically, the gene sequence of interest is linked in-frame to the 3'-end side of the base sequence that encodes the signal peptide or the functional fragment thereof in the target exon sequence of the endogenous gene. Accordingly, a fusion gene encoding a fusion polypeptide is constructed at the gene locus of the endogenous gene, in which the N-terminus of the protein of interest or a fragment thereof is fused to the C-terminal side of the signal peptide of the endogenous gene or a functional fragment thereof, and which comprises the signal peptide of the endogenous gene or a functional fragment thereof and the protein of interest or a fragment thereof. In this fusion polypeptide, the signal peptide or a functional fragment thereof and the protein of interest or a fragment thereof may be directly linked, or an amino acid sequence other than the signal peptide encoded by the target exon sequence (for example, an amino acid sequence located at the N-terminus in the mature protein described later) may be inserted between the signal peptide or a functional fragment thereof and the protein of interest or a fragment thereof.

In one embodiment, the endogenous gene encodes a protein that constitutes silk thread. The protein that constitutes silk thread is not particularly limited and may be, for example, fibroin, sericin, and/or fibrohexamerin. The fibroin may be a fibroin H chain and/or a fibroin L chain. The sericin is not particularly limited and may be, for example, sericin 1.

In the case of the fibroin H chain of the silkworm, the precursor protein comprising the signal peptide consists of the amino acid sequence shown in SEQ ID NO: 1, and precursor protein lacking the signal peptide consists of the amino acid sequence shown in SEQ ID NO: 2. The signal peptide of the fibroin H chain consists of the amino acid sequence from positions 1 to 21 in SEQ ID NO: 1.

In the fibroin H chain gene of the silkworm, the signal peptide is encoded by the first and second exons, and the C-terminal amino acid residue of the signal peptide is encoded by the second exon (Figure 2B). In the genome sequence of the fibroin H chain gene shown in SEQ ID NO: 3, the first exon is from position 1001 to 1042, the first intron is from position 1043 to 2013, the second exon comprises the positions 2014 to at least 17763, and the region encoding the signal peptide in the second exon is from position 2014 to 2034.

In the case of the fibroin L chain of the silkworm, the precursor protein comprising the signal peptide consists of the amino acid sequence shown in SEQ ID NO: 4, and precursor protein lacking the signal peptide consists of the amino acid sequence shown in SEQ ID NO: 5. The signal peptide of the fibroin L chain consists of the amino acid sequence from positions 1 to 16 in SEQ ID NO: 4.

In the fibroin L chain gene of the silkworm, the signal peptide is encoded by the first, second, and third exons, and the C-terminal amino acid residue of the signal peptide is encoded by the third exon (Figure 2C). In the genome sequence shown in SEQ ID NO: 6, the first exon of the fibroin L chain gene is from position 574 to 889, the first intron is from position 890 to 966, the second exon is from position 967 to 1036, the second intron is from position 1037 to 8976, the third exon is from position 8977 to 9059, and the region encoding the signal peptide in the third exon is from position 8977 to 8988.

In the case of sericin 1 of the silkworm, a plurality of isoforms are generated by alternative splicing. In the case of an example of the isoforms, the precursor protein comprising the signal peptide consists of the amino acid sequence shown in SEQ ID NO: 7, and precursor protein lacking the signal peptide consists of the amino acid sequence shown in SEQ ID NO: 8. The signal peptide of above-mentioned isoform of sericin 1 consists of the amino acid sequence from positions 1 to 19 in SEQ ID NO: 7.

In the sericin 1 gene of the silkworm, the signal peptide of above-mentioned isoform is encoded by the first and second exons, and the C-terminal amino acid residue of the signal peptide is encoded by the second exon (Figure 2A). In the genome sequence shown in SEQ ID NO: 9, in the sericin 1 gene, the first exon of the above-mentioned isoform is from position 947 to 1039, the first intron is from position 1040 to 3051, the second exon is from position 3052 to 3082, and the region encoding the signal peptide in the second exon is from position 3052 to 3069.

In the case of fibrohexamerin of the silkworm, the precursor protein comprising the signal peptide consists of the amino acid sequence shown in SEQ ID NO: 10, and precursor protein lacking the signal peptide consists of the amino acid sequence shown in SEQ ID NO: 11. The signal peptide of fibrohexamerin consists of the amino acid sequence from positions 1 to 17 in SEQ ID NO: 10.

In the fibrohexamerin gene of the silkworm, the signal peptide is encoded by the first exon, and the C-terminal amino acid residue of the signal peptide is encoded by the first exon. In the genome sequence shown in SEQ ID NO: 12, the first exon of the fibrohexamerin gene is from position 918 to 1052, the first intron is from position 1053 to 1536, the second exon is from position 1537 to 1756, and the region encoding the signal peptide in the first exon is from position 1001 to 1051.

In the transgenic lepidopteran insect of the present invention, the gene sequence of interest may be introduced into a single endogenous gene or into a plurality of endogenous genes. Furthermore, the transgenic lepidopteran insect of the present invention may heterozygously have or homozygously comprise the exon sequence comprising the gene sequence of interest. When the gene sequence of interest is introduced into a plurality of endogenous genes, the types of gene sequences of interest introduced into the plurality of endogenous genes may be the same or different.

In one embodiment, the plurality of endogenous genes into which the gene sequence of interest is introduced may be the genes encoding the fibroin H chain and the fibroin L chain; the genes encoding the fibroin H chain and sericin 1; or the genes encoding the fibroin H chain, the fibroin L chain, and sericin 1.

In one embodiment, in the transgenic lepidopteran insect of the present invention, the gene sequence of interest has a stop codon. In a further embodiment, in the transgenic lepidopteran insect of the present invention, the target exon sequence comprises a transcription terminator sequence on the 3'-end side of the stop codon of the gene sequence of interest.

In another embodiment, the gene sequence of interest does not have a stop codon. In this embodiment, the protein of interest or a fragment thereof is fused between the signal peptide or a functional fragment thereof and a mature protein (a protein formed by cleaving and removing the signal peptide from a precursor protein) encoded by the endogenous gene or a C-terminal fragment thereof (for example, a fragment in which a part of the N-terminal sequence encoded by the target exon sequence in the mature protein is deleted). As a result, the N-terminus of the protein of interest or a fragment thereof is fused to the C-terminal side of the signal peptide of the endogenous gene or a functional fragment thereof, and the C-terminus of the protein of interest or a fragment thereof is fused to the N-terminal side of the mature protein or a C-terminal fragment thereof. Therefore, a fusion gene is constructed at the gene locus of the endogenous gene, which encodes a fusion protein comprising the signal peptide of the endogenous gene or a functional fragment thereof, the protein of interest or a fragment thereof, and the mature protein or a C-terminal fragment thereof.

In one embodiment, the protein of interest is a fluorescent protein, an antibody, an antigenic polypeptide, an enzyme, a cytokine, or an antimicrobial polypeptide. For example, when the protein of interest is an antibody, the heavy chain gene and the light chain gene that constitute the antibody may be introduced into distinct endogenous genes.

### 1-4. Effect

The transgenic lepidopteran insect of the present invention can produce stably and in large quantities a protein of interest encoded by a gene of interest introduced into a target exon sequence.

In the conventional GAL4/UAS system, the GAL4 gene and the UAS regulatory sequence are introduced at random positions on the genome, which can cause large fluctuations in the expression level of the protein of interest. In contrast, in the transgenic lepidopteran insect of the present invention, the gene of interest can be expressed by utilizing the native promoter and enhancer activities of the endogenous gene, thereby allowing more reliable control of its expression level.

### 2. Double-stranded circular DNA

### 2-1. Outline

The second aspect of the present invention is a double-stranded circular DNA. According to the double-stranded circular DNA of this aspect, a gene sequence of interest can be introduced into a target exon sequence located on the 3'-end side of a genome cleavage position within an intron sequence in an endogenous gene of a lepidopteran insect. The double-stranded circular DNA of this aspect can be used, for example, for the knock-in of a gene of interest based on the TAL-PITCh (Precise Integration into Target Chromosome) method.

### 2-2. Definition

In this aspect, a "double-stranded circular DNA" means a circular, double-stranded DNA molecule that comprises at least a gene sequence of interest for the purpose of introducing the gene sequence of interest into an endogenous gene of a lepidopteran insect. The double-stranded circular DNA is preferably a vector that can be maintained and/or replicated within bacterial cells such as *Escherichia coli,* and can, for example, comprise sequences necessary for maintenance and replication in the cell (such as an origin of replication and/or a gene encoding an antibiotic resistance protein). The double-stranded circular DNA may be, for example, a plasmid vector.

As used herein, "genome editing" is a gene targeting technology that performs insertion of a foreign gene (knock-in) or disruption of a target gene (knock-out) at an arbitrary position on a genome by utilizing DNA repair mechanisms associated with a double-strand break (DSB) by a DNA-cleaving enzyme. Known genome editing technologies include the zinc finger nuclease (ZFN) method, the TALEN method, and the CRISPR/Cas method, and any of these methods may be used herein.

The "TALEN (Transcription Activator-Like Effector Nuclease) method" is a genome editing technology that employs an artificial DNA-cleaving enzyme obtained by fusing a TAL effector (TALE) protein derived from *Xanthomonas* spp., a plant pathogenic bacterium, and a non-specific endonuclease domain. TALEN is a protein consisting of a TALE domain that comprises repeats of a DNA-binding unit as a DNA-binding domain, and a non-specific endonuclease domain such as the nuclease domain of FokI. Of these, the nuclease domain having the enzyme activity to cleave DNA functions as a dimer. Thus, TALEN functions as a dimer consisting of a polypeptide that recognizes the DNA sequence upstream (on the 5' side) and proximal to the double-strand break (DSB) site in the target base sequence (often herein referred to as "Left-TALEN") and a polypeptide that recognizes the DNA sequence downstream (on the 3' side) and proximal to the DSB site (often herein referred to as "Right-TALEN"). The DNA-binding unit that constitutes the TALE domain has variations at the 12th and 13th amino acid residues from the N-terminal side, and as a pair of two amino acids, can specifically recognize each of the four types of bases that constitute DNA (A: adenine, G: guanine, C: cytosine, T: thymine). For example, an adenine is recognized when the amino acid residues at positions 12 and 13 are N-I or N-N; a guanine when the amino acid residues at positions 12 and 13 are N-N; a cytosine when the amino acid residues at positions 12 and 13 are H-D; and a thymine when the amino acid residues at positions 12 and 13 are N-G. The number of repeats of the DNA-binding unit can be varied according to the base length of the target base sequence. By manipulating the TALE domain, gene targeting to any DNA sequence in the genome can be achieved. The method of gene knock-out using the TALEN method in lepidopteran insects such as silkworms is a known technology. For example, the method described in Takasu Y., et al., 2013, PLoS One 8, e73458 may be referred to.

The "Zinc-Finger Nuclease (ZFN) method" is a genome editing technology that employs an artificial DNA-cleaving enzyme consisting of a zinc-finger domain as a DNA-binding domain and a non-specific endonuclease domain such as the nuclease domain of FokI. One zinc-finger motif can recognize three bases and bind to a target nucleic acid. Therefore, linking a plurality of zinc-finger motifs together allows for the specific recognition and binding to bases with a length three times the number of the linked motifs. ZFN functions as a dimer, and after binding to the target site, its endonuclease activity introduces a double-strand break (DSB) at a specific site in the target nucleic acid.

The "CRISPR/Cas (Clustered Regularly Interspaced Short Palindromic Repeats / CRISPR associated proteins) method" is a genome editing technology that utilizes an acquired immune system that has evolved in bacteria and archaea to eliminate foreign DNA or RNA such as that from viruses and plasmids. Variations have been reported, including the CRISPR/Cas9 method that uses the Cas9 protein, as well as others that use other Cas proteins such as Cpf1 and Cas13a. Bacteria and archaea fragment invading foreign DNA or RNA and insert the fragments into the CRISPR region in their genome, which then serve as templates for the synthesis of approximately 40-bp CRISPR RNAs (crRNAs). The crRNA, either directly or via a trans-activating RNA (tracrRNA), binds with a Cas protein having the nuclease activity to form a CRISPR/Cas complex. The CRISPR/Cas complex, via the crRNA, binds to and cleaves a target DNA or RNA sequence that has a base sequence complementary to the crRNA. When a double-strand nuclease such as Cas9 or Cpf1 is used as the Cas protein, a DSB is induced at the target site.

As used herein, a "genome editing enzyme" means a protein having the activity to specifically cleave and edit a target site in a genome. Examples of the genome editing protein include TALEN (Transcription activator-like effector nuclease), Cas9 (CRISPR associated protein 9), and ZFN (zinc finger nuclease), which can be used for the genome editing described above. When the genome editing protein is TALEN, Left TALEN and Right TALEN can be used as TALEN that can function as a dimer. When the genome editing protein is Cas9, a guide RNA such as crRNA as described above is necessary to perform genome editing.

### 2-3. Configuration

The double-stranded circular DNA of this aspect comprises a first recognition sequence, a second spacer sequence, a first spacer sequence, a genome homologous sequence, and a gene sequence of interest, in this order. Here, the first recognition sequence, the second spacer sequence, the first spacer sequence, and the genome homologous sequence are derived from the base sequence of a genomic region comprising an endogenous gene that serves as a target for introducing a gene sequence of interest. It should be noted that the double-stranded circular DNA of this aspect comprises a second recognition sequence on the 5'-end side in the genome homologous sequence.

In the introduction of a gene of interest using the double-stranded circular DNA of this aspect, a double-strand break site is generated in an intron sequence on a genome using two genome editing enzymes (hereinafter referred to as "first genome editing enzyme" and "second genome editing enzyme") in the target endogenous gene. In this specification, this double-strand break site is referred to as "genome cleavage position." The genome cleavage position can be set at any position within an intron sequence of the endogenous gene, and is preferably placed at a position other than functional sequences necessary for splicing, such as the splice donor sequence, the splice acceptor sequence, and the branch site. It should be noted that, depending on the type of the genome editing enzyme, the genome cleavage position may not be identified with precision. Even in such cases, when designing the double-stranded circular DNA of the present invention, each element sequence constituting the double-stranded circular DNA is specified based on the position assumed as the genome cleavage position. The position where the two above-mentioned genome editing enzymes actually cleave the genome and the double-stranded circular DNA is not limited to that position, and may be a position in its vicinity (for example, any position in the first spacer sequence and/or the second spacer sequence).

The first recognition sequence, the second spacer sequence, and the first spacer sequence contained in the double-stranded circular DNA of this aspect, as well as the second recognition sequence located on the 5'-end side of the genome homologous sequence, are derived from a base sequence located in the vicinity of the genome cleavage position in the genome sequence of the endogenous gene. The "first recognition sequence" and the "second recognition sequence" are identical to the base sequences that are located on the 5'-end side and the 3'-end side, respectively, of the genome cleavage position in the genome sequence of the endogenous gene, and are recognized and bound by the first genome editing enzyme and the second genome editing enzyme, respectively. The base length of the first recognition sequence and the second recognition sequence varies depending on the type of the genome editing enzyme, and is usually 8 to 30 bases in length, for example, 10 to 25 bases in length, 12 to 20 bases in length, or 14 to 18 bases in length. The "first spacer sequence" is derived from a sequence adjacent to the 5' end of the genome cleavage position in the genome sequence of the endogenous gene, and is derived from the base sequence located between the above-mentioned first recognition sequence and the genome cleavage position. The "second spacer sequence" is derived from a sequence adjacent to the 3' end of the genome cleavage position in the genome sequence of the endogenous gene, and is derived from the base sequence located between the genome cleavage position and the above-mentioned second recognition sequence. The base length of the first spacer sequence and the second spacer sequence varies depending on the type of the genome editing enzyme, and is usually 6 to 30 bases in length, for example, 8 to 25 bases in length, 10 to 20 bases in length, or 12 to 15 bases in length. Here, the endogenous gene comprises the first recognition sequence, the first spacer sequence, the second spacer sequence, and the second recognition sequence in order from the upstream side of the gene, whereas the double-stranded circular DNA of this aspect is characterized by that the arrangement of the first spacer sequence and the second spacer sequence is reversed.

The genome homologous sequence contained in the double-stranded circular DNA of this aspect comprises the above-mentioned second recognition sequence on its 5'-end side, and consists of a base sequence homologous to the genome sequence from the second recognition sequence to an exon sequence or a partial sequence thereof located on the 3'-end side of the intron sequence comprising the genome cleavage position. Here, the exon sequence or a partial sequence thereof located on the 3'-end side of the intron sequence comprising the genome cleavage position may be an exon sequence adjacent to the 3'-end side of the intron sequence comprising the genome cleavage position. In the double-stranded circular DNA of this aspect, the exon sequence or a partial sequence thereof contained on the 3'-end side of the genome homologous sequence encodes the C-terminal side of the signal peptide or a functional fragment thereof, and is linked in-frame to the gene sequence of interest located further on the 3'-end side of the exon sequence or a partial sequence thereof. In the double-stranded circular DNA of this aspect, the genome homologous sequence is not particularly limited as long as it comprises the second recognition sequence of the genome editing enzyme. The base length of the genome homologous sequence may be, for example, 15 to 20,000 bases in length, 20 to 10,000 bases in length, 50 to 5,000 bases in length, 100 to 2,000 bases in length, or 500 to 1,000 bases in length.

In one embodiment, in the double-stranded circular DNA of this aspect, the region from the first recognition sequence to the second recognition sequence in the genome homologous sequence consists of the first recognition sequence, the second spacer sequence, the first spacer sequence, and the second recognition sequence in the genome homologous sequence.

In one embodiment, the gene sequence of interest in the double-stranded circular DNA of this aspect comprises a stop codon. In a further embodiment, the double-stranded circular DNA of this aspect comprises a transcription terminator sequence on the 3'-end side of the gene sequence of interest.

In one embodiment, the double-stranded circular DNA of this aspect comprises a marker gene for identifying individuals in which the gene sequence of interest has been introduced into an endogenous gene. For example, the marker gene can be placed further on the 3'-end side of a transcription terminator sequence that is placed on the 3'-end side of the gene sequence of interest.

The type of the genome editing enzyme that recognizes the first recognition sequence and the second recognition sequence contained in the double-stranded circular DNA of this aspect is not limited, and may be TALEN, ZFN, and/or Cas9. For example, the genome editing enzyme that recognizes the first recognition sequence and the second recognition sequence may be TALEN. In this case, the two genome editing enzymes that recognize the first recognition sequence and the second recognition sequence may be Left-TALEN and Right-TALEN that function as a dimer.

In one embodiment, in the endogenous gene in this aspect, a plurality of exons including the first exon encode the signal peptide.

### 2-4. Effect

Introduction of the double-stranded circular DNA of this aspect together with the first and second genome editing enzymes into an egg of a lepidopteran insect allows for microhomology-mediated end-joining between the first spacer sequence adjacent to the genome cleavage position on the genome and the first spacer sequence in the double-stranded circular DNA. This makes it possible to insert a gene sequence of interest into a target exon sequence located on the 3'-end side of the genome cleavage position within an intron sequence in an endogenous gene of the lepidopteran insect.

In one embodiment, the double-stranded circular DNA of this aspect can be used for the TAL-PITCh method. For the TAL-PITCh method, a known technical document (Nature communications, 2014, 5:5560) can be referred to.

### 3. Donor nucleic acid

### 3-1. Outline

A third aspect of the present invention is a donor nucleic acid. According to the donor nucleic acid of this aspect, a gene sequence of interest can be introduced into a target exon sequence located on the 3'-end side or the 5'-end side of a genome cleavage position within an intron sequence in an endogenous gene of a lepidopteran insect. The donor nucleic acid of this aspect can be used, for example, for the knock-in of a gene of interest based on the homologous recombination method.

### 3-2. Configuration

As used herein, a "donor nucleic acid" refers to a nucleic acid for introducing a gene sequence of interest into an endogenous gene of a lepidopteran insect. The form of the donor nucleic acid is not limited, and it may be, for example, a double-stranded circular DNA such as a plasmid vector, or a linear DNA.

The donor nucleic acid of this aspect comprises a first genome homologous sequence and a second genome homologous sequence, as well as a gene sequence of interest arranged between them. The first genome homologous sequence and the second genome homologous sequence are derived from the base sequence of a genomic region comprising the target endogenous gene into which the gene sequence of interest is to be introduced.

In the introduction of a gene of interest using the donor nucleic acid of this aspect, in order to generate a double-strand break site within an intron sequence on the genome in the target endogenous gene, a genome editing enzyme that recognizes a sequence within a region proximal to the double-strand break site. As in the second aspect, this double-strand break site is referred to as "genome cleavage position" in this aspect. It should be noted that, as described above, depending on the type of the genome editing enzyme, the genome cleavage position may not be identified with precision. Even in such cases, when designing the donor nucleic acid of the present invention, each element sequence constituting the donor nucleic acid is specified based on the position assumed as the genome cleavage position. The position where the above-mentioned genome editing enzymes actually cleave the genome is not limited to that position, and may be a position in its vicinity. The genome cleavage position can be set at any position within an intron sequence of the endogenous gene, and is preferably placed at a position other than sequences necessary for splicing, such as the splice donor sequence, the splice acceptor sequence, and the branch site. Furthermore, in this aspect, this sequence recognized by the genome editing enzyme is referred to as "recognition sequence of the genome editing enzyme" or simply "recognition sequence."

The specific configuration of the first genome homologous sequence and the second genome homologous sequence contained in the donor nucleic acid of this aspect differs between the cases where the genome cleavage position is located on the 5'-end side of the target exon sequence into which the gene sequence of interest is to be introduced, and where it is located on the 3'-end side of the genome cleavage position, and will therefore be described separately below. (1) Embodiment in which the genome cleavage position is located on the 5'-end side of the target exon sequence

In an embodiment where the genome cleavage position is located on the 5'-end side of the target exon sequence, the first genome homologous sequence consists of a base sequence homologous to the genome sequence from a base located on the 5'-end side of the genome cleavage position on the genome (for example, a base located 10 bases, 20 bases, 50 bases, 100 bases, 500 bases, or 1,000 bases or more upstream of the genome cleavage position) to the target exon sequence or a partial sequence thereof that is located on (or adjacent to) the 3'-end side of the intron sequence comprising the genome cleavage position. In this embodiment, the genome sequence corresponding to the first genome homologous sequence has a mutation in its recognition sequence so as not to be cleaved by the above-mentioned genome editing enzyme that cleaves the genome cleavage position. The type of the mutation is not particularly limited, and may be, for example, a substitution, deletion, and/or insertion of a base within the recognition sequence. The number of mutated bases within the recognition sequence is not particularly limited. For example, one or a plurality of bases may be substituted, deleted, and/or inserted, and more specifically, one or more, two or more, three or more, four or more, five or more, or six or more bases may be substituted, deleted, and/or inserted.

In this embodiment, the second genome homologous sequence consists of a base sequence homologous to a genome sequence located on the 3'-end side of the target sequence or a partial sequence thereof.

In this embodiment, the base length of the first and second genome homologous sequences is not particularly limited, and may be, for example, 100 to 20,000 bases in length, 200 to 10,000 bases in length, 500 to 5,000 bases in length, or 1,000 to 2,000 bases in length. (2) Embodiment in which the genome cleavage position is located on the 3'-end side of the target exon sequence

In an embodiment where the genome cleavage position is located on the 3'-end side of the target exon sequence, the first genome homologous sequence consists of a base sequence homologous to the genome sequence from a base located on the 5'-end side of the intron sequence comprising the genome cleavage position (specifically, a base on the 5'-end side of the position in the target exon sequence to which the gene sequence of interest is to be inserted, for example, a base located 100 bases, 200 bases, 500 bases, 1,000 bases, 5,000 bases, or 10,000 bases or more upstream of the position to which the gene sequence of interest is to be inserted) to the target exon sequence or a partial sequence thereof located on (or adjacent to) the 5'-end side of the intron sequence (for example, up to the base encoding the C-terminal amino acid residue of the signal peptide or a functional fragment thereof in the target exon sequence, or up to a base encoding an amino acid residue further on the C-terminal side than the C-terminal amino acid residue of the signal peptide or a functional fragment thereof in the target exon sequence).

In this embodiment, the second genome homologous sequence consists of a base sequence homologous to the genome sequence from a base located on the 3'-end side of the target exon sequence or a partial sequence thereof and on the 5'-end side of the genome cleavage position (for example, a base encoding an amino acid residue further on the C-terminal side than the C-terminal amino acid residue of the signal peptide or a functional fragment thereof in the target exon sequence) to a base located on the 3'-end side of the genome cleavage position (for example, a base located 10 bases, 20 bases, 50 bases, 100 bases, 500 bases, 1,000 bases, 5,000 bases, or 10,000 bases or more downstream of the genome cleavage position). In this embodiment, the genome sequence corresponding to the second genome homologous sequence has a mutation in its recognition sequence so as not to be cleaved by the above-mentioned genome editing enzyme that cleaves the genome cleavage position. The type of the mutation and the number of mutated bases are not particularly limited, and may be, for example, a substitution, deletion, and/or insertion of one or a plurality of bases within the recognition sequence, similar to (1) above.

In this embodiment, the base length of the first and second genome homologous sequences is not particularly limited, and may be, for example, 100 to 20,000 bases in length, 200 to 10,000 bases in length, 500 to 5,000 bases in length, or 1,000 to 2,000 bases in length.

In the donor nucleic acid of this aspect, the target exon sequence or a partial sequence thereof contained in either the first or the second genome homologous sequence encodes the signal peptide or a functional fragment thereof or a sequence on the C-terminal side of the signal peptide or a functional fragment thereof, and is linked in-frame to the gene sequence of interest located further on the 3'-end side of the exon sequence or a partial sequence thereof, optionally via a base sequence encoding a plurality of amino acid residues.

In one embodiment, the gene sequence of interest in the donor nucleic acid of this aspect comprises a stop codon. In a further embodiment, the donor nucleic acid of this aspect comprises a transcription terminator sequence on the 3'-end side of the gene sequence of interest.

In one embodiment, the donor nucleic acid of this aspect comprises a marker gene for identifying a transgenic lepidopteran insect in which the gene sequence of interest has been introduced into an endogenous gene.

The type of the genome editing enzyme used in the homologous recombination method using the donor nucleic acid of this aspect is not limited, and may be TALEN, ZFN, and/or Cas9.

In one embodiment, the donor nucleic acid of this aspect comprises a nuclease recognition sequence at an end of the first genome homologous sequence and/or the second genome homologous sequence opposite to the gene sequence of interest. The nuclease recognition sequence is not particularly limited, and may be the above-mentioned recognition sequence of the genome editing enzyme that cleaves the genome cleavage position, or may be a restriction enzyme recognition sequence that can be cleaved by any restriction enzyme different from that genome editing enzyme. In the case where the nuclease recognition sequence is a TALEN recognition sequence, the nuclease recognition sequence may be a combination of two recognition sequences recognized by Left TALEN and Right TALEN.

### 3-3. Effect

Introduction of the donor nucleic acid of this aspect together with a genome editing enzyme into an egg of a lepidopteran insect can induce homologous recombination in an endogenous gene of the lepidopteran insect to insert a gene sequence of interest into a target exon sequence.

### 4. Method for producing a transgenic lepidopteran insect

### 4-1. Outline

A fourth aspect of the present invention is a method for producing a transgenic lepidopteran insect. The production method of this aspect allows for the introduction of the double-stranded circular DNA described in the second aspect or the donor nucleic acid described in the third aspect into an egg of a lepidopteran insect by a microinjection method, thereby introducing a gene sequence of interest into an exon sequence of an endogenous gene and producing a transgenic lepidopteran insect.

### 4-2. Method

The production method of this aspect comprises, as an essential step, an introduction step of introducing a double-stranded circular DNA or a donor nucleic acid into an egg of a lepidopteran insect by a microinjection method, and comprises, as optional steps, an egg acquisition step and a transgenic lepidopteran insect selection step. The configuration of each step will be described below.

### (1) Egg acquisition step

The "egg acquisition step" is a step of obtaining eggs after having a female parent adult silkworm lay eggs. The method for acquiring the eggs may be carried out by a conventional method in the art. Oviposition is initiated by giving a silkworm egg board to a mated female parent silkworm. The temperature during oviposition is 23 to 28°C, preferably around 25°C. Typically, a female silkworm begins oviposition several hours after mating. In order for the DNA introduced into the eggs to be incorporated into the nucleus, it is necessary to perform microinjection within a period of 2 to 8 hours, preferably 3 to 6 hours, after oviposition.

### (2) Introduction step

The "introduction step" is a step of introducing a double-stranded circular DNA or a donor nucleic acid into an egg of a lepidopteran insect by a microinjection method.

In an embodiment where the introduction step in the production method of this aspect introduces a double-stranded circular DNA into an egg, the configuration of the double-stranded circular DNA is in accordance with the description of the second aspect. In the introduction step of this embodiment, the double-stranded circular DNA described in the second aspect, the first genome editing enzyme described in the second aspect or a nucleic acid encoding the first genome editing enzyme in a state which allows for its expression, and the second genome editing enzyme described in the second aspect or a nucleic acid encoding the second genome editing enzyme in a state which allows for its expression are introduced into an egg of a lepidopteran insect by a microinjection method.

In an embodiment where the introduction step in the production method of this aspect introduces a donor nucleic acid into an egg, the configuration of the donor nucleic acid is in accordance with the description of the third aspect. In the introduction step of this embodiment, the donor nucleic acid described in the third aspect, and the genome editing enzyme described in the third aspect or a nucleic acid that encodes the genome editing enzyme in a state which allows for its expression are introduced into an egg of a lepidopteran insect by a microinjection method.

As used herein, "in a state which allows for its expression" refers to a state where a gene to be expressed is arranged in a region downstream of a promoter that is under the control of the promoter. Furthermore, the nucleic acid that encodes the genome editing enzyme in a state which allows for its expression may be an RNA such as mRNA, or a DNA such as a plasmid DNA or a linear DNA. A DNA that encodes the genome editing enzyme in a state which allows for its expression comprises a promoter that can be expressed in an egg of a lepidopteran insect in addition to the base sequence encoding the first or second genome editing enzyme, and may optionally comprise components such as a marker gene (selection marker), an enhancer, a terminator, an origin of replication, and a poly(A) signal.

The microinjection method may be carried out by a method known in the art. For example, an injection solution is prepared by dissolving or diluting the double-stranded circular DNA or the donor nucleic acid, and the genome editing enzyme or a nucleic acid that encodes the genome editing enzyme in a state which allows for its expression, with a solvent such as water or a buffer to an appropriate concentration. Subsequently, microinjection is performed on fertilized eggs 3 to 6 hours after oviposition. The amount of nucleic acid to be introduced is not particularly limited. The amount may be appropriately determined according to the type, properties, and purpose of the nucleic acid. Usually, 50 nL to 30 nL is sufficient. The lepidopteran insect eggs after introduction may be incubated until hatching under appropriate conditions, for example, at 25°C.

### (3) Transgenic lepidopteran insect selection step

The "transgenic lepidopteran insect selection step" is a step of selecting a transgenic lepidopteran insect from the hatched lepidopteran insects. This step may also be carried out by a method known in the art. For example, when the double-stranded circular DNA or the donor nucleic acid used in the introduction step comprises a marker gene, the desired transgenic lepidopteran insect can be easily selected based on the expression of that marker gene.

As used herein, a "marker gene" is a polynucleotide consisting of a base sequence that encodes a marker protein, also called a selection marker.

As used herein, a "marker protein" is a protein that can impart a new trait that is not found in the host lepidopteran insect via the expression of the marker gene, and examples include enzymes, fluorescent proteins, pigment-synthesizing proteins, and luminescent proteins. Transformants that carry the introduced nucleic acid can be readily identified based on the activity of the marker protein.

### 5. Method for producing a protein of interest or a fusion protein

### 5-1. Outline

A fifth aspect of the present invention is a method for producing a protein of interest or a fragment thereof, or a fusion protein comprising the protein of interest or a fragment thereof. According to the production method of this aspect, a protein of interest or a fragment thereof, or a fusion protein comprising the protein of interest or a fragment thereof can be produced in large quantities using the transgenic lepidopteran insect of the first aspect or a transgenic lepidopteran insect produced by the production method described in the fourth aspect.

### 5-2. Production method

The production method of the present invention comprises a rearing step and a collection step. Each step will be described below.

### (1) Rearing step

The "rearing step" is a step of rearing the transgenic lepidopteran insect of the first aspect or a transgenic lepidopteran insect produced by the production method described in the fourth aspect. Regarding the method for rearing the transgenic lepidopteran insects, each may be reared by a technique known in the art for that insect. For example, if the lepidopteran insect is a silkworm, reference may be made to "Sanshu Soron; authored by Takeo Takami, published by National Silkworm Seed Association." The diet may be natural leaves of larval host tree species, such as leaves of the genus *Morus* for *Bombyx mori* or *Bombyx mandarina*; leaves of *Ricinus communis* or *Ailanthus altissima* for *Samia cynthia ricini;* or leaves of the family *Fagaceae* for *Antheraea pernyi,* or may be an artificial diet, such as Silkmate L4M or Purebred Silkworm Diet for 1st-3rd instars (Nosan Corporation). An artificial diet is preferable in view of the fact that it can suppress the occurrence of disease, enables feeding of stable quality and quantity, and allows for aseptic rearing if necessary. A simple rearing method will be described below using silkworms as an example.

Hakitate (first feeding to newly hatched larva) is performed with eggs laid by an appropriate number (e.g., 4 to 10) of females of the same line of transgenic lepidopteran insects. The hatched larvae are transferred from the paper for oviposition to a container lined with damp proof paper (paraffin-coated paper), which serves as a rearing bed, and are fed by arranging an artificial diet such as Silkmate on the damp proof paper. In principle, the feed is changed once for each of the 1st and 2nd instars, and one to three times for the 3rd instar. If there is a large amount of uneaten old feed, it is removed to prevent spoilage. For rearing 4th- to 5th-instar grown silkworms, the silkworms are transferred to a large container, and the number of the silkworm per container is adjusted appropriately. Depending on the humidity and the condition inside the container, the container may be covered with a lid made of damp proof paper, acrylic, or mesh. The rearing temperature is maintained at 25 to 28°C throughout all instars.

### (2) Collection step

The "collection step" is a step of collecting the protein of interest or a fragment thereof, or a fusion protein comprising the protein of interest or a fragment thereof, which has been expressed in the silk gland cells by the transgenic lepidopteran insect larva, secreted, and subsequently accumulated in the lumen of the silk gland.

The transgenic lepidopteran insect used in this aspect expresses in the silk gland cells a precursor protein in which a signal peptide or a functional fragment thereof is fused to the N-terminal side of the protein of interest or a fragment thereof, or a fusion protein comprising the protein of interest or a fragment thereof and a mature protein or a C-terminal fragment thereof encoded by an endogenous gene on the C-terminal side of the protein of interest or a fragment thereof (hereinafter referred to as "protein of interest or the like"). The precursor protein expressed in the silk gland cells is transported to the endoplasmic reticulum by the action of the signal peptide or a functional fragment thereof. After the signal peptide or a functional fragment thereof is cleaved by the action of an enzyme such as a peptidase present in the endoplasmic reticulum, the protein of interest or the like is secreted into the lumen of the silk gland. The protein of interest or the like, after the signal peptide or a functional fragment thereof has been cleaved, is secreted and spun out of the body from the anterior silk gland during the pupation period. Therefore, methods for collecting the protein of interest or the like include a method of collecting from the cocoons, or a method for direct collection after dissection of the silk glands from the insect body during the late final instar to the prepupal stage. In particular, the method for collection from the cocoons is superior in that the protein of interest or the like can be collected easily.

In the method for collecting the protein of interest or the like from the cocoons, first, larvae in the late final instar are subjected to mounting (Jozoku: moving larvae to a cocooning frame called "Mabushi") to allow them to perform cocooning. Next, the protein of interest or the like is extracted from the cocoons. The extraction method is not particularly limited. For example, the protein of interest or the like can be collected simply by immersing the cocoons in water or a suitable neutral extraction buffer that does not contain any agent for denaturing proteins (e.g., phosphate-buffered saline, pH 7.2, with or without 1% Tween-20 and 0.05% sodium azide). To enhance the extraction effect, the cocoons may be cut or crushed before immersion. The extraction temperature is a low temperature of 0 to 10°C, preferably 0 to 5°C, to prevent thermal denaturation of the protein of interest or the like. However, if the protein of interest or the like is not a heat-sensitive peptide, the extraction can also be performed at 10 to 40°C. The extract may be stirred as necessary. The extraction time varies depending on the extraction conditions, such as the state of the cocoons (e.g., whether cocoons are in an uncut state or in a powdered state), the volume of the extract, the extraction temperature, and the presence or absence of stirring, and thus may be appropriately determined according to the conditions. Insoluble components such as fibroin can be removed from the extract by centrifugation or filtration as necessary.

The method of collecting the protein of interest or the like by dissecting the silk glands from the insect bodies during the late final instar to the prepupal stage can be achieved by methods known in the art. For example, a silkworm immediately before spinning on the 6th day of the final (5th) instar may be anesthetized on ice, incised dorsally, and the silk glands dissected out with forceps without damaging the silk glands (see "New Biological Experiments Using Silkworms," edited by Yasushi Mori, Sanseido Co., Ltd., 1970, pp. 249-255). The dissected silk glands can be gently shaken, for example, in the aforementioned extraction buffer at a temperature of 0 to 10°C, preferably 0 to 5°C, to elute the protein of interest or the like into the buffer. If the protein of interest or the like is not a heat-sensitive peptide, the extraction may also be performed at a temperature of 10 to 40°C. Thereafter, contaminants such as tissue debris may be removed by centrifugation or filtration, and the supernatant containing the protein of interest or the like may be collected.

### 5-3. Effect

According to the production method of the present invention, use of the larva of the transgenic lepidopteran insect of the first aspect or a transgenic lepidopteran insect produced by the production method described in the fourth aspect as a protein production system allows for the production in large quantities and easy collection of the protein of interest or the like, as compared to the case of using a GAL4/UAS line.

### 6. Fusion protein

A sixth aspect of the present invention is a fusion protein. The fusion protein of this aspect comprises, in order from the N-terminal side, a protein of interest or a fragment thereof, and any protein that constitutes silk thread. The protein constituting silk thread contained in the fusion protein of this aspect may be fibroin, sericin, or fibrohexamerin. Furthermore, the fibroin may be a Fibroin H chain and/or a Fibroin L chain. Furthermore, the protein constituting silk thread contained in the fusion protein of this aspect may be a full-length mature protein.

The protein of interest or a fragment thereof contained in the fusion protein of this aspect is not particularly limited. For example, the protein of interest or a fragment thereof may be a fluorescent protein, an antibody, an antigenic polypeptide, an enzyme, a cytokine, or an antimicrobial polypeptide.

### 7. Cocoon or silk thread

A seventh aspect of the present invention is a cocoon or silk thread. The cocoon or silk thread of this aspect comprises the fusion protein of the sixth aspect. In a further embodiment, in the cocoon or silk thread of this aspect, any protein constituting silk thread consists of the fusion protein of the sixth aspect. For example, any one or more proteins selected from the group consisting of Fibroin H chain, Fibroin L chain, sericin 1, sericin 2, sericin 3, and fibrohexamerin, contained in the cocoon or silk thread of this aspect, consist of the fusion protein described in the sixth aspect. For example, in the case where Fibroin H chain consists of the fusion protein described in the sixth aspect, all Fibroin H chains in the cocoon or silk thread of this aspect are the fusion protein described in the sixth aspect, and proteins not fused with the protein of interest are substantially not included.

In one embodiment, the cocoon or silk thread of this aspect is derived from the transgenic lepidopteran insect of the first aspect or a transgenic lepidopteran insect produced by the production method described in the fourth aspect. This transgenic lepidopteran insect may homozygously comprise an exon sequence comprising the gene sequence of interest.

In the case where the fusion protein contained in the cocoon or silk thread of this aspect is a fluorescent protein fused with fibroin, fibrohexamerin, or the like, the cocoon or silk thread of this aspect has extremely strong fluorescence, and has an extremely strong coloration that is discernible to the naked eye, for example, even under normal white light. Since this coloration is overwhelmingly stronger than the coloration of fluorescent cocoons or fluorescent silk threads that can be produced with conventional technology, a material with high utility value can be provided.

### Examples

### <Example 1: Preparation of a knock-in line for production of a useful protein>

### (Purpose)

The GAL4/UAS system in the conventional art is a gene regulation system that utilizes a combination of the transcription factor GAL4, derived from yeast, and the regulatory sequence UAS. In the GAL4/UAS system used as a protein production system in silkworms, an expression system that expresses a protein of interest in silk glands or the like is constructed by crossing a GAL4 line, which expresses the GAL4 gene under the control of a promoter of a gene such as a silk gene, with a UAS line, which expresses the gene of interest under the control of the regulatory sequence UAS.

In the GAL4/UAS system, construct the expression system is time-consuming because it is necessary to separately establish the GAL4 line and the UAS line before crossing. Furthermore, since the GAL4 gene and the UAS regulatory sequence are introduced at random positions on the genome, the expression level of the protein of interest can fluctuate and is difficult to predict. Therefore, it is necessary to produce a large number of lines, examine the expression level in individuals obtained by crossing the GAL4 and UAS lines, and then select good parental lines based on the results.

Therefore, the present inventors conceived of constructing a new expression system for producing a protein of interest stably and in large quantities by knocking-in a gene sequence of interest into an endogenous gene. More specifically, when a gene of interest, which encodes a protein of interest designed to be fused with a signal peptide, is knocked-in into an exon sequence encoding the signal peptide in an endogenous sericin or fibroin gene, there is a possibility that the gene of interest can be highly expressed by utilizing the native promoter and enhancer activities of the endogenous gene.

However, efficient knock-in into an endogenous gene requires cleaving the target gene with a genome editing enzyme or the like.

The present inventors attempted a knock-in by designing a genome cleavage position within an exon sequence in Comparative Example 1 described later, which resulted in 95% or more of the individuals in the injected generation failing to form normal cocoons, and 98% or more of the individuals failing to develop into adults capable of mating. Therefore, it is extremely difficult to establish a line with this method.

Accordingly, in this Example, it was investigated whether the above-mentioned problems could be overcome by designing a genome cleavage position within an intron sequence and performing a knock-in into the exon sequence.

### (Methods and Results)

In this Example, a gene of interest that encodes the EGFP protein as the protein of interest to be fused on the C-terminal side of the signal peptide is knocked-in into an exon sequence that encodes the signal peptide in an endogenous silk gene. For the knock-in of the gene of interest, the TAL-PITCh (precise integration into target chromosome) method and the homologous recombination method are used, and an intron sequence adjacent to the 5'-end side of the target exon sequence is cleaved with the genome editing enzyme TALEN (transcription activator-like effector nuclease) (Figure 2). It should be noted that, this Example differs from Examples 5 to 6 described later in that the EGFP gene sequence as the gene of interest has a stop codon and a transcription terminator sequence on the 3'-end side (Figure 1).

Knock-in into the fibroin H (FibH) gene, the fibroin L (FibL) gene, and the sericin 1 (Ser1) gene was performed by the following methods. It should be noted that, in the following examples, the vectors for expressing TALENs were constructed with reference to Y. Takasu, S. Sajwan, T. Daimon, M. Osanai-Futahashi, K. Uchino, H. Sezutsu, T. Tamura, M. Zurovec (2013): Efficient TALEN construction for Bombyx mori gene targeting, PLoS One, 8, e73458. Furthermore, TALEN mRNA was synthesized using the mMESSAGE mMACHINE T7 ULTRA Transcription Kit (Invitrogen) with each TALEN expression vector as a template.

### (1) Knock-in into the fibroin H gene by the TAL-PITCh method

In the fibroin H gene (hereinafter referred to as "FibH gene"), the first exon and the second exon encode the signal peptide. The EGFP gene sequence was introduced into the second exon using the TAL-PITCh method such that the EGFP protein would be fused on the C-terminal side of the signal peptide of FibH.

Specifically, a position within the first intron between the first exon and the second exon in the genome sequence of the FibH gene (SEQ ID NO: 3) (a position between position 1945 and position 1964 in SEQ ID NO: 3; Figure 2A) was designed as the genome cleavage position. A double-stranded circular DNA shown in Figure 3B was constructed as a donor nucleic acid used for the TAL-PITCh method (hereinafter referred to as "SP(FibH)-EGFP donor nucleic acid for TAL-PITCh"). The SP(FibH)-EGFP donor nucleic acid for TAL-PITCh comprises, in this order, a first recognition sequence, a second spacer sequence, a first spacer sequence, a genome homologous sequence, a gene sequence of interest, a transcription terminator sequence, and a marker gene (Figure 3B).

Here, the first spacer sequence is adjacent to the 5'-end side of the genome cleavage position and is a 10-bp sequence consisting of positions 1945 to 1954 in SEQ ID NO: 3. The second spacer sequence is adjacent to the 3'-end side of the genome cleavage position and is a 10-bp sequence consisting of positions 1955 to 1964 in SEQ ID NO: 3. The first recognition sequence is recognized by Left TALEN on the 5'-end side of the first spacer sequence and is a 20-bp sequence consisting of positions 1925 to 1944 in SEQ ID NO: 3. The genome homologous sequence is a base sequence homologous to the genome sequence from the second recognition sequence to the codon encoding the C-terminal residue of the signal peptide in the second exon sequence, and is a 70-bp sequence consisting of positions 1965 to 2034 in SEQ ID NO: 3. Here, the second recognition sequence is recognized by Right TALEN on the 3'-end side of the second spacer sequence and is a 20-bp sequence consisting of positions 1965 to 1984 in SEQ ID NO: 3. The gene sequence of interest is the EGFP gene sequence, and as the transcription terminator sequence, the transcription terminator sequence of the sericin 1 gene derived from silkworms was used. The base sequence from the first recognition sequence to the transcription terminator sequence of the EGFP gene in the SP(FibH)-EGFP donor nucleic acid for TAL-PITCh is shown in SEQ ID NO: 13. Furthermore, the amino acid sequence of a protein in which the signal peptide derived from FibH is fused to the N-terminal side of the C-terminal fragment of EGFP excluding the start methionine, encoded by the post-knock-in gene (Figure 3C), is shown in SEQ ID NO: 14 (hereinafter referred to as "SP(FibH)-EGFP fusion protein").

The SP(FibH)-EGFP donor nucleic acid for TAL-PITCh was injected together with mRNAs encoding Left TALEN and Right TALEN, which were synthesized using the mMESSAGE mMACHINE T7 ULTRA Transcription Kit (Invitrogen), into silkworm eggs 2 to 8 hours after oviposition, which were from the w1-pnd line exhibiting white eyes, white eggs, and non-diapause, maintained at the National Agriculture and Food Research Organization. The eggs after injection were incubated under humidified conditions at 25°C until hatching. The injected generation of silkworms was crossed with the parental line, and the resulting next-generation larvae were selected based on the fluorescence of DsRed2 expressed throughout the body or EGFP expressed in the silk glands to obtain a knock-in silkworm line. Hereinafter, the obtained knock-in line is referred to as "SP(FibH)-EGFP knock-in line." Furthermore, the knocked-in Fib H gene is referred to as "SP(FibH)-EGFP knock-in gene."

Of the larvae that developed from the 105 embryos that underwent the above microinjection, 103 individuals achieved normal cocooning. Therefore, no defective cocoon formation was observed in the injected generation larvae.

Furthermore, among the adults of the injected generation that developed after normal cocooning, 34 individuals out of 36 female silkworms successfully mated with wild-type male silkworms and laid eggs, while 50 individuals out of 53 male silkworms successfully mated with wild-type female silkworms, resulting in the female silkworms laying eggs. Therefore, no defective mating was observed in the adults of the injected generation.

From the results above, it was demonstrated that by cleaving the genome within an intron sequence, knock-in lines capable of normal cocooning and mating can be produced with extremely high efficiency.

In the SP(FibH)-EGFP knock-in line, strong fluorescence of EGFP was observed in the middle and posterior silk glands from the 1st instar larvae. Figure 5 shows the results of observation of the silk glands and cocoons of 5th instar larvae. Specifically, larvae on the 6th day of the 5th instar, immediately before spinning, were anesthetized on ice, incised dorsally, and the middle and posterior silk glands dissected out with forceps without damaging the silk glands. As a result of observation with a fluorescence microscope without fixation, extremely strong EGFP fluorescence was observed (Figure 5, left side). Furthermore, the cocoons of this line exhibited a distinct yellow-green color under normal white light (Figure 5, right side).

### (2) Knock-in into the fibroin H gene by the homologous recombination method

The EGFP gene sequence was introduced into the second exon of the FibH gene using the homologous recombination method, such that the EGFP protein would be fused on the C-terminal side of the signal peptide of FibH.

Specifically, similarly to (1) above, a position within the first intron (a position between position 1945 and position 1964 in SEQ ID NO: 3; Figure 2A) was designed as the genome cleavage position. A double-stranded circular DNA shown in Figure 4 was constructed as a donor nucleic acid used for the homologous recombination method (hereinafter referred to as "SP(FibH)-EGFP donor nucleic acid for homologous recombination"). The SP(FibH)-EGFP donor nucleic acid for homologous recombination comprises a first genome homologous sequence and a second genome homologous sequence, as well as the EGFP gene sequence that is the gene sequence of interest, a transcription terminator sequence, and a marker gene arranged between the first and second genome homologous sequences, and comprises a TALEN recognition sequence at an end of the first genome homologous sequence and the second genome homologous sequence opposite to the EGFP gene sequence.

Here, the first genome homologous sequence is a 1,034-bp sequence that is homologous to the genome sequence from a base located on the 5'-end side of the genome cleavage position on the genome (position 1001 in SEQ ID NO: 3) to the codon encoding the C-terminal residue of the signal peptide in the second exon sequence. It should be noted that the first genome homologous sequence has a mutation so as not to be recognized by Left TALEN and Right TALEN described in (1) above in the vicinity of the above-mentioned genome cleavage position (specifically, the base sequence AACTTCGATTGAATGTGCGAAATTTATAGCTCAATATTTTAGCACTTATCGTATTG ATTT (SEQ ID NO: 33) located at positions 1925 to 1984 in SEQ ID NO: 3 is substituted with the base sequence AtCTaCGATTGAAaGaGCGtAATTTATAGCTCAATATTTTAtGCtCaTAaCGTATTGATT T (SEQ ID NO: 34)). The second genome homologous sequence is a 377-bp sequence that is homologous to the genome sequence located on the 3'-end side of the codon encoding the C-terminal residue of the signal peptide in the second exon sequence on the genome. In the SP(Ser1)-EGFP donor nucleic acid for homologous recombination, the base sequence from the TALEN recognition sequence and the first genome homologous sequence to the second genome homologous sequence and the TALEN recognition sequence is shown in SEQ ID NO: 15. Furthermore, the protein, which is encoded by the knocked-in gene (Figure 4) and in which the signal peptide derived from FibH is fused to the N-terminal side of EGFP, consists of the amino acid sequence shown in SEQ ID NO: 14, similarly to (1) above (hereinafter, referred to as "SP(FibH)-EGFP fusion protein," similarly to (1) above).

The SP(FibH)-EGFP donor nucleic acid for homologous recombination was injected together with mRNAs encoding Left TALEN and Right TALEN, which were synthesized using the mMESSAGE mMACHINE T7 ULTRA Transcription Kit (Invitrogen), into silkworm eggs from the w1-pnd line 2 to 8 hours after oviposition. The eggs after injection were incubated under humidified conditions at 25°C until hatching. The injected generation of silkworms were crossed with the parental line, and the resulting next-generation larvae were selected based on the fluorescence of DsRed2 expressed throughout the body or EGFP expressed in the silk glands to obtain a knock-in silkworm line. Hereinafter, the obtained knock-in line is referred to as "SP(FibH)-EGFP knock-in line," similarly to (1) above.

In the individuals that developed from the embryos that underwent the above microinjection, no defective cocoon formation or defective mating was observed, similarly to (1) above. Therefore, it was demonstrated that even with the homologous recombination method, by cleaving the genome within an intron sequence, knock-in lines capable of normal cocooning and mating can be produced efficiently.

### (3) Knock-in into the fibroin L (FibL) gene by the homologous recombination method

The EGFP gene sequence was introduced into the third exon of the fibroin L (hereinafter, referred to as "FibL") gene using the homologous recombination method, such that the EGFP protein would be fused on the C-terminal side of the signal peptide of FibL.

Specifically, a position within the second intron of the FibL gene (a position between position 8937 and position 8954 in SEQ ID NO: 6) was designed as the genome cleavage position. A double-stranded circular DNA was constructed as a donor nucleic acid used for the homologous recombination method (hereinafter referred to as "SP(FibH)-EGFP donor nucleic acid for homologous recombination"). The SP(FibL)-EGFP donor nucleic acid for homologous recombination comprises a first genome homologous sequence and a second genome homologous sequence, as well as the EGFP gene sequence that is the gene sequence of interest, a transcription terminator sequence, and a marker gene arranged between the first and second genome homologous sequences, and comprises a TALEN recognition sequence at an end of the first genome homologous sequence and the second genome homologous sequence opposite to the EGFP gene sequence.

Here, the above-mentioned genome cleavage position is cleaved by Left TALEN that recognizes a 20-bp sequence consisting of positions 8917 to 8936 in SEQ ID NO: 6, and Right TALEN that recognizes a 20-bp sequence consisting of positions 8955 to 8974 in SEQ ID NO: 6.

Furthermore, the first genome homologous sequence is a 1128-bp sequence that is homologous to the genome sequence from a base located on the 5'-end side of the genome cleavage position on the genome (position 7864 in SEQ ID NO: 6) to the codon encoding the C-terminal residue of the signal peptide in the third exon sequence. It should be noted that the first genome homologous sequence has a mutation so as not to be recognized by Left TALEN and Right TALEN in the vicinity of the above-mentioned genome cleavage position (specifically, the base sequence CCCGAGAAAACAATTTGTTGTGTATAATTTAAACCAAAACCCGAATTTAATTTTTC GC (SEQ ID NO: 35) located at positions 8917 to 8974 in SEQ ID NO: 6 is substituted with the base sequence CCCGAGAAAAgAATTcGTTcTGTATAATTTAAACCAAAAttCGAATTTAATTTTTCGC (SEQ ID NO: 36)). The second genome homologous sequence is a 1362-bp sequence that is homologous to the genome sequence located on the 3'-end side of the codon encoding the C-terminal residue of the signal peptide in the third exon sequence on the genome. In the SP(FibL)-EGFP donor nucleic acid for homologous recombination, the base sequence from the TALEN recognition sequence and the first genome homologous sequence to the second genome homologous sequence and the TALEN recognition sequence is shown in SEQ ID NO: 16. Furthermore, the protein, which is encoded by the knocked-in gene and in which the signal peptide of FibL is fused to the N-terminal side of the C-terminal fragment of EGFP excluding the start methionine, consists of the amino acid sequence shown in SEQ ID NO: 17 (hereinafter referred to as "SP(FibL)-EGFP fusion protein").

The SP(FibL)-EGFP donor nucleic acid for homologous recombination was injected together with mRNAs encoding Left TALEN and Right TALEN, which were synthesized using the mMESSAGE mMACHINE T7 ULTRA Transcription Kit (Invitrogen), into silkworm eggs from the w1-pnd line 2 to 8 hours after oviposition. The eggs after injection were incubated under humidified conditions at 25°C until hatching. A knock-in silkworm line was obtained by the same method as in (2) above. Hereinafter, the obtained knock-in line is referred to as the "SP(FibL)-EGFP knock-in line," similarly to (1) above. Furthermore, the knocked-in FibL gene is referred to as "SP(FibL)-EGFP knock-in gene."

In the individuals that developed from the embryos that underwent the above microinjection, no defective cocoon formation or defective mating was observed, similarly to (1) above.

### (4) Knock-in into the sericin 1 gene by the homologous recombination method

The EGFP gene sequence was introduced into the second exon of the sericin 1 (hereinafter, referred to as "Ser1") gene using the homologous recombination method, such that the EGFP protein would be fused on the C-terminal side of the signal peptide of Ser1.

Specifically, a position within the first intron of the Ser1 gene (a position between position 3020 and position 3033 in SEQ ID NO: 9) was designed as the genome cleavage position. A double-stranded circular DNA was constructed as a donor nucleic acid used for the homologous recombination method (hereinafter referred to as "SP(Ser1)-EGFP donor nucleic acid for homologous recombination"). The SP(Ser1)-EGFP donor nucleic acid for homologous recombination comprises a first genome homologous sequence and a second genome homologous sequence, as well as the EGFP gene sequence that is the gene sequence of interest, a transcription terminator sequence, and a marker gene arranged between the first and second genome homologous sequences, and comprises a TALEN recognition sequence at an end of the first genome homologous sequence and the second genome homologous sequence opposite to the EGFP gene sequence.

Here, the above-mentioned genome cleavage position is cleaved by Left TALEN that recognizes a 19-bp sequence consisting of positions 3001 to 3019 in SEQ ID NO: 9, and Right TALEN that recognizes a 16-bp sequence consisting of positions 3034 to 3049 in SEQ ID NO: 9.

Furthermore, the first genome homologous sequence is a 2069-bp sequence that is homologous to the genome sequence from a base located on the 5'-end side of the genome cleavage position on the genome (position 947 in SEQ ID NO: 9) to the codon encoding the C-terminal residue of the signal peptide in the second exon sequence. It should be noted that the first genome homologous sequence has a mutation so as not to be recognized by Left TALEN and Right TALEN in the vicinity of the above-mentioned genome cleavage position (specifically, the base sequence TATATTGTAAAGCACAACATATATATTAATGAATTTTTTATTTATTTTTC (SEQ ID NO: 37) located at positions 3000 to 3049 in SEQ ID NO: 9 is substituted with the base sequence agTATTGagAAGCACAAgtaATATATTAATGAATTTTTTcTTTcTTTTTC (SEQ ID NO: 38)). The second genome homologous sequence is a 2000-bp sequence that is homologous to the genome sequence located on the 3'-end side of the codon encoding the C-terminal residue of the signal peptide in the second exon sequence on the genome. In the SP(Ser1)-EGFP donor nucleic acid for homologous recombination, the base sequence from the restriction enzyme recognition sequence and the first genome homologous sequence to the second genome homologous sequence and the restriction enzyme recognition sequence is shown in SEQ ID NO: 18. Furthermore, the protein, which is encoded by the knocked-in gene and in which the signal peptide of Ser1 is fused to the N-terminal side of the C-terminal fragment of EGFP excluding the start methionine, consists of the amino acid sequence shown in SEQ ID NO: 19 (hereinafter referred to as "SP(Ser1)-EGFP fusion protein").

The SP(Ser1)-EGFP donor nucleic acid for homologous recombination was injected together with mRNAs encoding Left TALEN and Right TALEN, which were synthesized using the mMESSAGE mMACHINE T7 ULTRA Transcription Kit (Invitrogen), into silkworm eggs from the w1-pnd line 2 to 8 hours after oviposition. The eggs after injection were incubated under humidified conditions at 25°C until hatching. A knock-in silkworm line was obtained by the same method as in (2) above. Hereinafter, the obtained knock-in line is referred to as the "SP(Ser1)-EGFP knock-in line," similarly to (1) above. Furthermore, the knocked-in Ser1 gene is referred to as "SP(Ser1)-EGFP knock-in gene."

In the individuals that developed from the embryos that underwent the above microinjection, no defective cocoon formation or defective mating was observed, similar to (1) above.

### <Example 2: Production of the EGFP protein>

### (Purpose)

The expression level of the EGFP protein in the silk glands of each knock-in line produced in Example 1 is measured.

### (Methods and Results)

### (1) Silkworm line

The knock-in lines produced in Example 1 (2) to (4) were crossed with each other to prepare lines having a combination of a plurality of knock-in genes. In this Example, for example, a line having two knock-in genes, the SP(FibH)-EGFP knock-in gene and the SP(FibL)-EGFP knock-in gene, is referred to as the SP(FibH)-EGFP/SP(FibL)-EGFP knock-in line. It should be noted that in the silkworms used in this Example, all knock-in genes are heterozygous.

In this Example, a line obtained by crossing the FibH+Ser1-GAL4 line in which the GAL4 gene was expressed under the control of the FibH gene promoter and the Ser1 gene promoter, with the UAS-EGFP line in which the EGFP gene was expressed under the control of the UAS regulatory sequence (hereinafter referred to as the "EGFP-producing GAL4/UAS line"), was used as a control group.

### (2) Rearing conditions

The rearing of silkworms was carried out by the following method. Larvae of all instars were reared in a rearing room at 25 to 27°C on an artificial diet (Silkmate Purebred silkworm Diet 1st-3rd instars S, Nosan Corporation). The artificial diet was changed every 2 to 3 days (Uchino K. et al., 2006, J Insect Biotechnol Sericol, 75:89-97).

### (3) Measurement of the expression level of the EGFP protein

Larvae on the 6th day of the 5th instar, immediately before spinning, were anesthetized on ice, incised dorsally, and the silk glands dissected out with forceps without damaging the silk glands. For the silk glands, the middle silk glands and posterior silk glands were dissected. The silk glands were each placed in 10 mL per gland of PBS (pH 7.2)/1% Tween 20/0.05% sodium azide, and water-soluble proteins were extracted by shaking at room temperature for 24 hours. The obtained water-soluble protein extract was centrifuged at 2,000 x g for 10 minutes, and the supernatant was collected. The concentration of the EGFP protein in the water-soluble proteins contained in the supernatant was measured by the ELISA method. Specifically, 100 µL of the supernatant was added to a 96-well plate coated with an anti-GFP antibody (Aves GFP-1010, Cosmo Bio), and left to stand at room temperature for 1 hour. After washing three times with PBS / 0.05% Tween 20, a horseradish peroxidase-conjugated anti-GFP antibody (Rockland Immunochemicals, Inc.) was added, and the plate was left to stand at room temperature for 1 hour. After washing three times with PBS / 0.05% Tween 20, development was performed using the TMB Peroxidase EIA Substrate Kit (Bio-Rad Laboratories, Inc.), and the reaction was stopped by adding 1N sulfuric acid. The color development was quantified with a plate reader (SpectraMax iD3; Molecular Devices, LLC.). A standard curve was created using serial dilutions (1 to 400 pg/µL) of a recombinant GFP protein (Takara Bio Inc.; Z2373N).

The results of measuring the EGFP expression level per silkworm are shown in Figure 6.

The EGFP expression level in the SP(Ser1)-EGFP knock-in line (Figure 6, SP(Ser1)-EGFP) slightly exceeded that of the EGFP-producing GAL4/UAS line (Figure 6, Control (GAL4/UAS)). In the EGFP-producing GAL4/UAS line, the GAL4 protein is expressed by two promoters, the Ser1 gene promoter and the FibH gene promoter. Of the 3.3 mg EGFP expression level, the expression level derived from the Ser1 gene promoter is estimated to be 1 mg or less. Therefore, the EGFP expression level of the SP(Ser1)-EGFP knock-in line is considered to be overwhelmingly larger.

The EGFP expression level of the SP(FibH)-EGFP knock-in line (Figure 6, SP(FibH)-EGFP) was twice or more that of the SP(FibL)-EGFP knock-in line (Figure 6, SP(FibL)-EGFP), and four times or more that of the SP(Ser1)-EGFP knock-in line (Figure 6, SP(Ser1)-EGFP). This result was unexpected, considering that the molar amounts of FibH protein and FibL protein produced in the silk gland of the silkworm are equal in accordance with the constitution of fibroin, and that the fibroin constituting silk thread is three times the weight of sericin.

The SP(FibH)-EGFP/SP(FibL)-EGFP/SP(Ser1)-EGFP knock-in line (Figure 6, rightmost) exhibited an EGFP expression level of 33.7 mg, greatly exceeding the amount expected as the sum of the EGFP expression levels in the SP(FibH)-EGFP knock-in line, the SP(FibL)-EGFP knock-in line, and the SP(Ser1)-EGFP knock-in line.

### <Example 3: Production of the GM-CSF protein>

### (Purpose)

A knock-in is performed by the homologous recombination method such that granulocyte-macrophage colony-stimulating factor (GM-CSF) is fused on the C-terminal side of the signal peptide of FibH. The GM-CSF production amount in the silk glands is evaluated.

### (Methods and Results)

### (1) Silkworm line

The homologous recombination method described in Example 1 (2) was carried out by replacing the gene of interest from the EGFP gene sequence to the GM-CSF gene sequence. The GM-CSF gene sequence consists of the base sequence shown in SEQ ID NO: 20 and encodes the GM-CSF protein consisting of the amino acid sequence shown in SEQ ID NO: 21. The obtained knock-in line is referred to as "SP(FibH)-GM CSF knock-in line." Furthermore, the protein, which is encoded by the knocked-in gene (Figure 7A) and in which the signal peptide derived from FibH is fused to the N-terminal side of the mature amino acid sequence of GM-CSF from which the GM-CSF-derived signal peptide has been removed, consists of the amino acid sequence shown in SEQ ID NO: 22 (hereinafter referred to as "SP(FibH)-GM-CSF fusion protein").

Furthermore, a line obtained by crossing the Ser1-GAL4 line in which the GAL4 gene was expressed under the control of the Ser1 gene promoter, with the UAS-GM-CSF line in which the GM-CSF gene was expressed under the control of the UAS regulatory sequence (hereinafter referred to as "middle silk gland GM-CSF-producing GAL4/UAS line"), and a line obtained by crossing the FibH-GAL4 line in which the GAL4 gene was expressed under the control of the FibH gene promoter, with the UAS-GM-CSF line (hereinafter referred to as "posterior silk gland GM-CSF-producing GAL4/UAS line") were used as control groups.

### (2) Western blotting

In accordance with the method described in Example 2, the middle and posterior silk glands were dissected from the lines, and the proteins in the silk glands were extracted. Subsequently, the undiluted or 2- to 128-fold diluted silk gland extract was mixed with specified amounts of NuPAGE LDS Sample Buffer (Thermo Fisher Scientific, Inc.) and NuPAGE Sample Reducing Agent (Thermo Fisher Scientific, Inc.) and heated at 70°C for 10 minutes to achieve SDS treatment. The SDS-treated samples were electrophoresed on an 8 cm × 13 cm 4-12% SDS-PAGE gel at a constant current of 20 mA for approximately 90 minutes. The gel was transferred to a PVDF membrane using a semi-dry transfer device (iBlot2, Thermo Fisher Scientific, Inc.). After transfer, the membrane was gently shaken in EZ wash (AE-1480, ATTO Corporation) for 5 minutes, and then reacted with the primary antibody (anti-GM-CSF antibody, 1:3000 dilution, from Immundiagnostik AG, product number AS1021.2) overnight at 4°C. After washing the membrane three times with EZ wash for 10 minutes each, the secondary antibody (Anti-Rabbit IgG, HRP-Linked Whole Ab Donkey, from Cytiva, product number NA934-100UL, 1:50,000 dilution) was allowed to react for 1 hour at room temperature. After washing the membrane three times with EZ wash for 10 minutes each, ECL prime (RPN2232, GE Healthcare) was allowed to react for 5 minutes, and the signal was detected with a Fusion FX (Vilber Bio Imaging).

The results are shown in Figure 7B. In the combined extract of the middle and posterior silk glands from the SP(FibH)-GM-CSF knock-in line, approximately 13 times and 3 times more GM-CSF was detected compared to the middle silk gland GM-CSF-producing GAL4/UAS line and the posterior silk gland GM-CSF-producing GAL4/UAS line, respectively. It was revealed that GM-CSF is expressed and secreted with extremely high efficiency in the silk glands of the SP(FibH)-GM-CSF knock-in line.

### <Example 4: Production of an antibody>

### (Purpose)

A knock-in is performed by the homologous recombination method such that the IgG H chain is fused on the C-terminal side of the signal peptide of FibH. Furthermore, a knock-in is performed by the homologous recombination method such that the IgG L chain is fused on the C-terminal side of the signal peptide of FibL. The obtained two knock-in lines were crossed to produce an antibody molecule comprising the IgG H chain and the IgG L chain.

### (Methods and Results)

### (1) Silkworm line

The homologous recombination method described in Example 1 (2) was carried out by replacing the gene of interest from the EGFP gene sequence to the IgG H chain gene sequence. The IgG H chain gene sequence consists of the base sequence shown in SEQ ID NO: 23 and encodes the IgG H chain consisting of the amino acid sequence shown in SEQ ID NO: 24. The obtained knock-in line is referred to as "SP(FibH)-IgG H chain knock-in line." Furthermore, the protein, which is encoded by the knocked-in gene (Figure 8A) and in which the signal peptide derived from FibH is fused to the N-terminal side of the IgG H chain, consists of the amino acid sequence shown in SEQ ID NO: 25 (hereinafter referred to as "SP(FibH)-IgG H chain fusion protein").

Furthermore, the homologous recombination method described in Example 1 (3) was carried out by replacing the gene of interest from the EGFP gene sequence to the IgG L chain gene sequence. The IgG L chain gene sequence consists of the base sequence shown in SEQ ID NO: 26 and encodes the IgG L chain consisting of the amino acid sequence shown in SEQ ID NO: 27. The obtained knock-in line is referred to as "SP(FibL)-IgG L chain knock-in line." Furthermore, the protein, which is encoded by the knocked-in gene (Figure 8B) and in which the signal peptide derived from FibL is fused to the N-terminal side of the IgG L chain, consists of the amino acid sequence shown in SEQ ID NO: 28 (hereinafter referred to as "SP(FibL)-IgG L chain fusion protein").

By crossing the two knock-in lines described above, a line was produced that had the two knock-in genes and was capable of producing IgG molecules comprising the IgG H chain and the IgG L chain (hereinafter referred to as "SP(FibH)-IgG H chain/SP(FibL)-IgG L chain knock-in line").

In this Example, a line obtained by crossing the FibH+Ser1-GAL4 line in which the GAL4 gene was expressed under the control of the FibH gene promoter and the Ser1 gene promoter, the UAS-IgG H chain line in which the IgG H chain gene was expressed under the control of the UAS regulatory sequence, and the UAS-IgG L chain line in which the IgG L chain gene was expressed under the control of the UAS regulatory sequence (hereinafter referred to as "antibody-producing GAL4/UAS line") was used as a control group.

### (2) Quantification of IgG expression level

In accordance with the method described in Example 2, the middle and posterior silk glands were dissected from the lines, and the proteins in the silk glands were extracted. Subsequently, IgG was purified using Ab SpinTrap (Cytiva), and the amount of IgG in the extract was quantified using Protein Assay BCA Kit (Nacalai Tesque, Inc.).

The results are shown in Figure 8C. It was demonstrated that the SP(FibH)-IgG H chain/SP(FibL)-IgG L chain knock-in line can produce approximately 4.6 times more IgG compared to the middle and posterior silk glands of the antibody-producing GAL4/UAS line. It was revealed that in the silk glands of the SP(FibH)-IgG H chain/SP(FibL)-IgG L chain knock-in line, antibody molecules comprising the IgG H chain and the IgG L chain are expressed and secreted with extremely high efficiency.

### <Example 5: Preparation of a knock-in line for production of a fusion protein>

### (Purpose)

In conventional technologies for producing a functional silk, such as a fluorescent silk comprising a silk protein fused with a fluorescent protein or a functional peptide, methods are known in which a repeat sequence portion located in the central part of FibH is replaced with a protein of interest, or in which a protein of interest is fused to the C-terminus of FibL. However, conventional technologies have had problems such as low expression levels and loss of activity of the protein of interest.

The present inventors conceived that knocking-in a gene of interest into an exon sequence that encodes a signal peptide in a silk gene, such as the sericin or fibroin gene, to fuse the protein of interest between the signal peptide and the mature protein formed by cleaving the signal peptide from the precursor protein encoded by the endogenous silk gene potentially enables the high expression of the silk protein fused with the protein of interest by utilizing the native promoter and enhancer activities of the endogenous silk gene.

Therefore, in this Example, a knock-in line that expresses a fusion silk protein will be produced by designing a genome cleavage position within an intron sequence located on the 5'-end side of an exon sequence encoding a signal peptide and applying the homologous recombination method similarly to Examples above.

### (Methods and Results)

In this Example, a gene of interest that encodes the EGFP protein as the protein of interest to be fused on the C-terminal side of the signal peptide is knocked-in into an exon sequence that encodes the signal peptide in an endogenous silk gene. For the knock-in of the gene of interest, the homologous recombination method is used, and an intron sequence adjacent to the 5'-end side of the target exon sequence is cleaved with the genome editing enzyme TALEN (Figure 10). It should be noted that this Example differs from Examples 1 to 4 described above in that the EGFP gene sequence that is the gene of interest does not contain a stop codon on the 3'-end side, and that the EGFP protein is linked in-frame with the silk protein (a mature protein formed by cleaving the signal peptide from a precursor protein encoded by the endogenous silk gene).

The donor nucleic acids used for the knock-in into the fibroin L (FibL) gene and the sericin 1 (Ser1) gene were prepared by the following methods.

### (1) EGFP-FibL donor nucleic acid

In the homologous recombination method described in Example 1 (3), the transcription terminator sequence and the marker gene were removed from the SP(FibL)-EGFP donor nucleic acid described in Example 1, and the EGFP gene sequence and the second genome homologous sequence were modified such that the C-terminal amino acid residue of the EGFP protein could be fused with the N-terminal amino acid residue of FibL after the signal peptide is cleaved, thereby constructing a donor nucleic acid (a double-stranded circular DNA) for homologous recombination (hereinafter referred to as "EGFP-FibL donor nucleic acid"). The configurations of the genome cleavage position in the endogenous FibL gene, Left TALEN and Right TALEN, the first genome homologous sequence, the TALEN recognition sequence, and the like were in accordance with Example 1. In the constructed donor nucleic acid, the base sequence from the TALEN recognition sequence and the first genome homologous sequence to the second genome homologous sequence and the restriction enzyme recognition sequence is the SP(FibH)-EGFP donor nucleic acid shown in SEQ ID NO: 15 from which the stop codon TAA at positions 1821 to 1823 is deleted. Furthermore, the EGFP-FibL precursor protein, which is encoded by the knocked-in gene and in which the signal peptide of FibL is fused to the N-terminal side of EGFP and the mature FibL protein after signal peptide cleavage is fused to the C-terminal side of EGFP, consists of the amino acid sequence shown in SEQ ID NO: 29. The EGFP-FibL mature protein, which is generated by cleaving the signal peptide from the EGFP-FibL precursor protein, consists of the amino acid sequence shown in SEQ ID NO: 30.

### (2) EGFP-Ser1 donor nucleic acid

In the homologous recombination method described in Example 1 (4), the transcription terminator sequence and the marker gene were removed from the SP(Ser1)-EGFP donor nucleic acid described in Example 1, and the EGFP gene sequence and the second genome homologous sequence were modified such that the C-terminal amino acid residue of the EGFP protein could be fused with the N-terminal amino acid residue of the mature Ser1 after the signal peptide is cleaved, thereby constructing a donor nucleic acid (a double-stranded circular DNA) for homologous recombination (hereinafter referred to as "EGFP-Ser1 donor nucleic acid"). The configurations of the genome cleavage position in the endogenous Ser1 gene, Left TALEN and Right TALEN, the first genome homologous sequence, the TALEN recognition sequence, and the like were in accordance with Example 1. In the constructed donor nucleic acid, the base sequence from the restriction enzyme recognition sequence and the first genome homologous sequence to the second genome homologous sequence and the restriction enzyme recognition sequence is the SP(Ser1)-EGFP donor nucleic acid shown in SEQ ID NO: 18 from which the stop codon TAA at positions 2841 to 2843 is deleted. Furthermore, the EGFP-Ser1 precursor protein, which is encoded by the knocked-in gene and in which the signal peptide of Ser1 is fused to the N-terminal side of EGFP and the mature Ser1 protein after signal peptide cleavage is fused to the C-terminal side of EGFP, consists of the amino acid sequence shown in SEQ ID NO: 31. The EGFP-Ser1 mature protein, which is generated by cleaving the signal peptide from the EGFP-Ser1 precursor protein, consists of the amino acid sequence shown in SEQ ID NO: 32.

The donor nucleic acids prepared in (1) and (2) above were injected into silkworm eggs together with mRNAs encoding TALENs in accordance with the methods described in Example 1 (3) and (4) to produce knock-in lines. Hereinafter, the knock-in lines are referred to as "EGFP-FibL knock-in line" and "EGFP-Serl knock-in line," respectively. Furthermore, the knocked-in FibL and Ser1 genes are referred to as "EGFP-FibL knock-in gene" and "EGFP-Ser1 knock-in gene," respectively.

In the individuals that developed from the embryos that underwent the above microinjection, no defective cocoon formation or defective mating was observed.

### <Example 6: Production of a fusion protein comprising the EGFP protein and a silk protein>

### (Purpose)

A fusion protein comprising the EGFP protein and a silk protein is produced using the knock-in lines produced in Example 5.

### (Methods and Results)

In accordance with the method described in Example 2, the middle and posterior silk glands were dissected from the knock-in lines produced in Example 5, and proteins in the silk glands were extracted. Subsequently, Western blotting was performed in accordance with the method described in Example 3 (2). In this Example, Anti-GFP Antibody Peroxidase Conjugate (1:4000 dilution, from Rockland Immunochemicals, Inc., product number 600-103-215) was used as the detection antibody. Furthermore, in this Example, the SP(Ser1)-EGFP knock-in line produced in Example 1 (4) and the w1-pnd line were used as control groups.

The results of the Western blotting are shown in Figure 11. In the EGFP-FibL knock-in line, the EGFP-FibL mature protein was detected (Figure 11A). In the EGFP-Ser1 knock-in line, the EGFP-Ser1 mature protein was detected (Figure 11B).

### <Example 7: Homozygote for knock-in gene>

### (Purpose)

An individual homozygously comprising the EGFP-FibL knock-in gene is produced and allowed to spin a cocoon.

### (Methods and Results)

The EGFP-FibL knock-in lines heterozygously comprising the EGFP-FibL knock-in gene were crossed with each other to produce an individual homozygously comprising the EGFP-FibL knock-in gene. Figure 12 shows photographs taken under normal white light of cocoons obtained by allowing the produced homozygotes and heterozygotes to spin cocoons. The homozygote exhibited a very vivid yellow-green color.

It is known that fluorescent cocoons produced using the piggyBac system by the method described in a reference (Insect Biochemistry and Molecular Biology, 2005, 35: 51-59) have the strongest fluorescence among conventional technologies. Figure 13 shows the results of observation under white light or fluorescence imaging of cocoons from wild-type silkworms, FibL-fused EGFP cocoons produced using the piggyBac system, and cocoons produced by the above-described heterozygotes and homozygotes, arranged side-by-side. For the fluorescence imaging, fluorescence was detected using an EGFP filter upon excitation with blue light. It was revealed that the heterozygotes of the EGFP-FibL knock-in line produced by the method of the present invention showed strong fluorescence, and the homozygotes showed overwhelmingly strong fluorescence compared to the FibL-fused EGFP cocoons produced using the piggyBac system.

### <Comparative Example 1: Production of a knock-in line where a genome cleavage position is designed within an exon sequence>

### (Purpose)

The EGFP gene is knocked-in into the fibroin H gene by a homologous recombination method, wherein a genome cleavage position is provided within an exon sequence rather than within an intron sequence. Comparison of its efficiency of producing the knock-in line is made with the case where the genome is cleaved within an intron sequence.

### (Methods and Results)

The homologous recombination method described in Example 1 (2) was modified such that the genome cleavage position was set within the second exon of the FibH gene, and the knock-in of the EGFP gene sequence into the fibroin H gene was performed. Specifically, a mutation was introduced into the donor nucleic acid for homologous recombination in the vicinity of the genome cleavage position within the second exon so that the donor nucleic acid would not be recognized by the TALEN. The above-mentioned donor nucleic acid for homologous recombination was injected together with mRNAs encoding TALENs, which were synthesized using the mMESSAGE mMACHINE T7 ULTRA Transcription Kit (Invitrogen), into 384 silkworm eggs, into silkworm eggs from the w1-pnd line 2 to 8 hours after oviposition. The eggs after injection were incubated under humidified conditions at 25°C until hatching. The injected generation of silkworms was reared to adulthood and allowed them to mate with the parental line to determine their mating ability.

The results are shown in Figure 14B. The homologous recombination method where the genome was cleaved within an exon sequence resulted in 113 out of 118 individuals that had grown to 5th instar larvae showing pupation failure, naked pupae, or thin cocoons, and exhibiting mating failure even upon eclosion, with only 5 individuals achieving production of normal cocoons. However, among the 5 normal cocoons, only 2 individuals were capable of mating; only 1 out of 2 females and only 1 out of 3 males had mating ability. The reason for the abnormalities occurring when an exon sequence is cleaved is thought to be due to the possibility that mutations such as frameshifts may be introduced at the genome cleavage position in the injected embryos, resulting in the failure of expression of a normally functioning protein.

This result is in contrast to the result in Example 1 (2), where almost no defective cocoon formation or defective mating was observed when the genome was cleaved within an intron sequence (Figure 14A), indicating that the method for producing a knock-in silkworm line by the method described in Example 1 can produce knock-in lines with overwhelmingly high efficiency. This can be because the introduction of minor mutations into intron sequences would have only a negligible effect on the expression of the normal protein.

### Industrial Applicability

According to the present invention, a protein of interest can be produced stably and in large quantities in a lepidopteran insect such as silkworms.

All publications, patents, and patent applications cited herein are incorporated herein by reference in their entirety.

## Claims

1. A transgenic lepidopteran insect, comprising a gene sequence of interest encoding a protein of interest or a fragment thereof in an exon sequence encoding a signal peptide or a functional fragment thereof of an endogenous gene,
wherein the protein of interest or the fragment thereof is fused between the signal peptide or the functional fragment thereof and a mature protein formed by cleaving the signal peptide from a precursor protein encoded by the endogenous gene or a C-terminal fragment thereof.

2. The transgenic lepidopteran insect of claim 1, wherein the mature protein is fibroin, sericin, and/or fibrohexamerin.

3. The transgenic lepidopteran insect of claim 2, wherein the fibroin is a fibroin H chain and/or a fibroin L chain.

4. The transgenic lepidopteran insect of claim 1, wherein the protein of interest is selected from the group consisting of a fluorescent protein, an antibody, an antigenic polypeptide, an enzyme, a cytokine, and an antimicrobial polypeptide.

5. The transgenic lepidopteran insect of claim 1, which homozygously comprises the exon sequence comprising the gene sequence of interest.

6. A donor nucleic acid for producing a transgenic lepidopteran insect using a homologous recombination method, wherein
the homologous recombination method comprises cleaving a genome cleavage position in an intron sequence in an endogenous gene by a genome editing enzyme,
the donor nucleic acid comprises
(a) a first genome homologous sequence and a second genome homologous sequence, derived from the endogenous gene, and
(b) a gene sequence of interest placed therebetween,
the first genome homologous sequence consists of a base sequence homologous to a genome sequence from a base positioned at the 5' end side of the genome cleavage position to an exon sequence positioned at the 3' end side of the intron sequence or a partial sequence thereof in the genome, and has a mutation in a recognition sequence of the genome editing enzyme,
the second genome homologous sequence consists of a base sequence homologous to a genome sequence positioned at the 3' end side of the exon sequence or the partial sequence thereof in the genome, and
the gene sequence of interest encodes a protein of interest or a fragment thereof which is fused between the signal peptide or the functional fragment thereof of the endogenous gene and a mature protein formed by cleaving the signal peptide from a precursor protein encoded by the endogenous gene or a C-terminal fragment thereof.

7. A donor nucleic acid for producing a transgenic lepidopteran insect using a homologous recombination method, wherein
the homologous recombination method comprises cleaving a genome cleavage position in an intron sequence in an endogenous gene by a genome editing enzyme,
the donor nucleic acid comprises
(a) a first genome homologous sequence and a second genome homologous sequence, derived from the endogenous gene, and
(b) a gene sequence of interest placed therebetween,
the first genome homologous sequence consists of a base sequence homologous to a genome sequence from a base positioned at the 5' end side of the intron sequence to an exon sequence positioned at the 5' end side of the intron sequence or a partial sequence thereof,
the second genome homologous sequence consists of a base sequence homologous to a genome sequence from a base positioned at the 3' end side of the exon sequence or the partial sequence thereof and at the 5' end side of the genome cleavage position to a base positioned at the 3' end side of the genome cleavage position, and has a mutation in a recognition sequence of the genome editing enzyme, and
the gene sequence of interest encodes a protein of interest or a fragment thereof which is fused between the signal peptide or the functional fragment thereof of the endogenous gene and a mature protein formed by cleaving the signal peptide from a precursor protein encoded by the endogenous gene or a C-terminal fragment thereof.

8. The donor nucleic acid of claim 6 or 7, comprising a nuclease recognition sequence at an end of the first genome homologous sequence and/or the second genome homologous sequence opposite to the gene sequence of interest.

9. The donor nucleic acid of claim 8, wherein the nuclease recognition sequence is a recognition sequence of the genome editing enzyme or a restriction enzyme recognition sequence.

10. A method for producing a transgenic lepidopteran insect, comprising:
an introduction step of introducing
a donor nucleic acid of claim 6 or 7, and
the genome editing enzyme or a nucleic acid encoding the genome editing enzyme in a state which allows for its expression,
into an egg of a lepidopteran insect by a microinjection method.

11. A method for producing a fusion protein comprising the protein of interest or the fragment thereof and the mature protein or the C-terminal fragment thereof, using the transgenic lepidopteran insect of any one of claims 1 to 5.

12. The method of claim 11, wherein the lepidopteran insect is a silk spinning insect, and the fusion protein is produced in the silk gland of the silk spinning insect.

13. A fusion protein comprising:
a protein of interest or a fragment thereof; and
fibroin, sericin, or fibrohexamerin,
in this order from the N-terminal side.

14. The fusion protein of claim 13, wherein the fibroin is a fibroin H chain and/or a fibroin L chain.

15. A cocoon or silk thread comprising the fusion protein of claim 13.

16. A cocoon or silk thread, wherein one or more silk proteins selected from the group consisting of fibroin H chain, fibroin L chain, sericin 1, sericin 2, sericin 3, and fibrohexamerin contained in the cocoon or silk thread consist of the fusion protein of claim 13.

17. The cocoon or silk thread of claim 16, which is derived from the transgenic lepidopteran insect of any one of claims 1 to 5.

18. The cocoon or silk thread of claim 16, wherein the protein of interest is selected from the group consisting of a fluorescent protein, an antibody, an antigenic polypeptide, an enzyme, a cytokine, and an antimicrobial polypeptide.
